# EUROPEAN PATENT SPECIFICATION

(11) **EP 2 063 909 B1**
(45) Date of publication and mention of the grant of the patent: **24.10.2012**
(21) Application number: 07838433.6
(22) Date of filing: 18.09.2007
(51) Int. Cl.: A61K 38/24, A61P 5/14, A61P 35/00

(54) **Formulations for therapeutic administration of thyroid stimulating hormone (TSH)**
Formulierungen zur therapeutischen Verabreichung schilddrüsenstimulierender Hormone (TSH)
Formulations pour administration thérapeutique de l'hormone stimulant la thyroïde (TSH)

(30) Priority: 19.09.2006 US 846077 P
(43) Date of publication of application: 03.06.2009
(73) Proprietor: Genzyme Corporation, Cambridge, MA 02142 (US)
(72) Inventor: CLARK, Eliana, Boylston, MA 01505 (US); MAGNER, James, Woodbridge, CT 06525 (US); SKELL, Jeffrey, Westborough, MA 01581 (US)
(74) Representative: Gates, Marie Christina Esther
(86) International application number: PCT/US2007/020221
(87) International publication number: WO 2008/036271

(56) References cited:
- EP-A- 1 291 021
- SUH ET AL: "Regulation of the phosphatidylinositol 3-kinase, Akt/protein kinase B, FRAP/mammalian target of rapamycin, and ribosomal S6 kinase 1 signaling pathways by thyroid-stimulating hormone (TSH) and stimulating type TSH receptor antibodies in the thyroid gland" THE JOURNAL OF BIOLOGICAL CHEMISTRY, vol. 278, 2003, pages 21960-21971, XP002468675
- MORELLE ET AL: "Characterization of N-glycans of recombinant human thyrotropin using mass spectrometry" RAPID COMMUNICATIONS IN MASS SPECTROMETRY, vol. 20, December 2005 (2005-12), pages 331-345, XP002454381
- MICHAILOVA ET AL: "Influence of aqueous medium on viscoelastic properties of carboxymethylcellulose sodium, hydroxypropy[i]methyl cellulose, and thermally pre-gelatinized starch gels" COLLOIDS AND SURFACES A: PHYSICO- CHEMICAL AND ENGINEERING ASPECTS, vol. 149, 1999, pages 515-520, XP002468716
- SOH ET AL: "Thyroid-stimulating hormone promotes the secretion of vascular endothelial growth factor in thyroid cancer cell lines" SURGERY, vol. 120, 1996, pages 944-947, XP005437680
- SZKUDLINSKI ET AL: "Subunit-specific functions of N-linked oligosaccharides in human thyrotropin: Role of terminal residues of alpha- and beta-subunit oligosaccharides in metabolic clearance and bioactivity", PROCEEDINGS OF THE NATIONAL ACADEMY OF SCIENCES, U.S.A., vol. 92, 1995, pages 9062-9066, XP001091168,
- DOELKER: "Cellulose derivatives", ADVANCES IN POLYMER SCIENCE, vol. 107, 1993, pages 199-265, XP009030076,
- KAMIDE: "Cellulose and cellulose derivatives", 2005, Elsevier ISBN: 978-0-444-02254-3
- MEYER ET AL: "Raman spectroscopic surface characterization of cellulose derivatives", FRESENIUS´ JOURNAL OF ANALYTICAL CHEMISTRY, vol. 370, 2001, pages 789-791,
- KAMEL ET AL: "Pharmaceutical significance of cellulose: A review", EXPRESS POLYMER LETTERS, vol. 2, 2008, pages 758-778,
- BAUMGARTNER ET AL: "Network structure of cellulose ethers used in pharmaceutical applications during swelling and at equilibrium", PHARMACEUTICAL RESEARCH, vol. 19, 2002, pages 1084-1090,
- 'Cellulose', [Online] Wikipedia (retrieved in 2011)

## Description

### FIELD OF THE INVENTION

This disclosure generally relates to novel formulations containing the active ingredient Thyroid Stimulating Hormone (TSH) having modified pharmacokinetic profiles as compared to prior art formulations.

### BACKGROUND OF THE INVENTION

Goiter is an enlargement of a thyroid gland. Symptoms of goiter include thyroid enlargement, neck fullness, breathing difficulties, coughing; wheezing, swallowing difficulties, neck vein distention, and dizziness. Over time, goiter can cause hypothyroidism as normal thyroid tissue is destroyed by an autoimmune or other thyroid disease. Alternatively, a goiter can progress to a toxic nodular goiter. In this case, the toxic nodular goiter can produce additional thyroid hormone and the patient can develop hyperthyroidism. Also, thyroid enlargement or development of hardened nodules from goiter can develop into thyroid malignancy, or thyroid cancer can develop without pre-existing goiter. Administration of TSH to patients can play a role in the diagnostic or therapeutic approach for various thyroid diseases, including goiter and thyroid cancer. For these diseases, the pharmacokinetic profile of the administered TSH may be important for the optimal success of the diagnostic or therapeutic procedures.

Currently, treatment of goiter includes administration of radioactive iodine or surgical removal of part or all of the thyroid gland. A side effect from radioactive iodine treatment is life-long hypothyroidism requiring daily treatment with a thyroid hormone. Surgical removal of the thyroid gland can also result in life-long hypothyroidism. Furthermore, there are risks associated with surgery, including injury to structures near the thyroid gland.

Goiter can occur when a thyroid gland is not able to produce enough thyroid hormone to meet a body's need. Other goiter patients can have an overactive thyroid gland.

Hypothyroidism is a condition in which a body lacks sufficient thyroid hormone and slow metabolism is usually the hallmark of the condition. Clinical symptoms include fatigue, weakness, weight gain, dry hair, dry skin, hair loss, cold intolerance, muscle cramps, constipation, depression, irritability, memory loss, and abnormal menstrual cycles. A positive diagnosis can be made with a blood test showing elevated levels of TSH, where normal levels of TSH are generally between about 0.4 to about 4.5 mIU/L. In a sample study of about 17,000 patients reflecting the U.S. population, about 4.6% reported hypothyroidism.

Hyperthyroidism is a condition caused by the effects of too much thyroid hormone on tissues of the body. Clinical symptoms include palpitations, heat intolerance, nervousness, insomnia, breathlessness, increased bowel movements, light or absent menstrual periods, and fatigue. Hyperthyroidism can be diagnosed with a blood test to measure the level of TSH. A low blood TSH strongly suggests that the thyroid is overproducing hormone on its own, where normal levels of TSH are generally between about 0.4 to about 4.5 mIU/L. Based on a sample study, there is a reported overall prevalence of hyperthyroidism in the range between 0.5 and 6.3% of the general population.

Thyroid cancer is a collection of diseases in which there is uncontrolled growth of cells derived from the thyroid. Thyroid cancer commonly has been classified as differentiated thyroid cancer, including papillary, follicular and Hurthle cell cancer, and other thyroid cancers, including medullary and anaplastic cancer. Over time, some differentiated thyroid cancers become less well differentiated, and may be classified as de-differentiated or poorly-differentiated cancer. Administration of TSH to patients with various types of thyroid cancer can play a role in diagnosis or therapy of thyroid cancer. The precise pharmacokinetics of TSH may be important for optimizing diagnostic and/or therapeutic procedures.

The suitability of cellulose polymers to influence the kinetic pattern of drug release was reported in Michailova *et al* (1999). "Neutral and semi-synthetic h) drophilic polymers are widely used in pharmaceutical technology to formulate controlled release drug delivery systems. The behaviour of the gel layer, formed around the hydrophilic matrices after water uptake, is of major importance for the drug release profiles" (abstract).

Recombinant human TSH is currently being clinically tested as a diagnostic agent in patients with thyroid carcinoma and recently it has been demonstrated that the use of rhTSH in humans is an effective method to stimulate 131I uptake without the disadvantages of hypothyroidism. The terminal residues on the α- and β-subunits comprising the herterodimeric glycoprotein, TSH, have been shown to have important functions in regulating the intrinsic activity and rate of clearance of the hormone from the circulation (Szkudlinski et al. PNAS. 1995). The authors report, "Present studies of phTSH and rhTSH subunit hybrids indicated that the N-linked carbohydrate residues of the 3 subunit play a more important role than those of the a subunit in determining MCR, which has a major impact on in vivo bioactivity. Moreover, our data demonstrate that terminal sialic acid residues on the a but not on the 13 subunit attenuate hTSH intrinsic potency, further emphasizing a distinct role of terminal sialylation in each subunit."

### SUMMARY OF THE INVENTION

This disclosure generally relates to novel formulations containing the active 5 ingredient TSH having modified pharmacokinetic profiles as compared to prior art formulations. In the following, the polymer is always a cellulose derivative.

Present invention provides a pharmaceutical composition comprising TSH and a pharmaceutically acceptable polymer that allows modified release of the TSH into a bloodstream of a patient, wherein the polymer is a cellulose derivative and when administered to the patient, the pharmaceutical composition provides an effective Tₘₐₓ of TSH in a serum of the patient that is at least about 20% longer than an effective Tₘₐₓ of TSH in the serum of the patient when a corresponding aqueous solution of TSH is administered.

One embodiment provides a method for providing a modified-release formulation of TSH comprising admixing an effective amount of T5H and an effective amount of a pharmaceutically-acceptable polymer, thereby providing a modified-release formulation.

Also disclosed is a method for treating a thyroid condition in a patient in need thereof, comprising delivering to the patient an effective amount of a pharmaceutical composition comprising an effective amount of TSH and an effective amount of a pharmaceutically-acceptable polymer.

Also disclosed is a method for maintaining a blood plasma concentration of TSH above 2.0 mIU/L in a patient suffering from a thyroid condition, comprising administering to the patient an effective amount of a pharmaceutical composition comprising an effective amount of TSH and an effective amount of a pharmaceutically-acceptable polymer, wherein the blood serum or plasma concentration of TSH is maintained above about 2.0 mIU/L for longer than about six hours after administration.

One embodiment provides a pharmaceutical composition comprising TSH and a pharmaceutically-acceptable polymer that allows modified release of the TSH into a bloodstream of a patient, wherein, when administered to the patient, the pharmaceutical dosage form provides an effective Tₘₐₓ of TSH in the patient's serum that is at least about 20% longer than the effective Tₘₐₓ of TSH in the patient's serum when a corresponding aqueous solution of TSH is administered.

One embodiment provides a pharmaceutical composition comprising TSH and a pharmaceutically-acceptable polymer that allows modified release of the TSH into a bloodstream of a patient, wherein, when administered to the patient, the pharmaceutical dosage form provides an effective Cₘₐₓ of TSH in the patient's serum that is at least about 20% lower than the effective Cₘₐₓ of TSH in the patient's serum when a corresponding aqueous solution of TSH is administered.

One embodiment provides a pharmaceutical composition comprising an effective amount of TSH and an effective amount of a pharmaceutically-acceptable polymer, wherein the composition has a viscosity of at least about 40 cps at room temperature.

One embodiment provides a pharmaceutical composition comprising an effective amount of TSH and an effective amount of a pharmaceutically-acceptable polymer, wherein, after administration to a patient in need thereof, the composition provides an effective Tₘₐₓ of at least six hours.

One embodiment provides a pharmaceutical composition comprising an effective amount of TSH and an effective amount of a pharmaceutically-acceptable polymer, wherein, after administration to a patient in need thereof, the composition provides a serum T₃ level of no more than 2.5 ng/ml over a 48-hour period.

One embodiment provides a pharmaceutical composition comprising an effective amount of TSH and an effective amount of a pharmaceutically-acceptable polymer, wherein, after administration to a patient in need thereof, the composition provides an effective Cₘₐₓ in the patient's serum of greater than about 2.0 mIU/L.

One embodiment provides a use of the pharmaceutical compositions of the disclosed embodiments for the manufacture of a medicament for treating a thyroid condition (e.g., goiter, thyroid cancer).

### BRIEF DESCRIPTION OF THE DRAWINGS

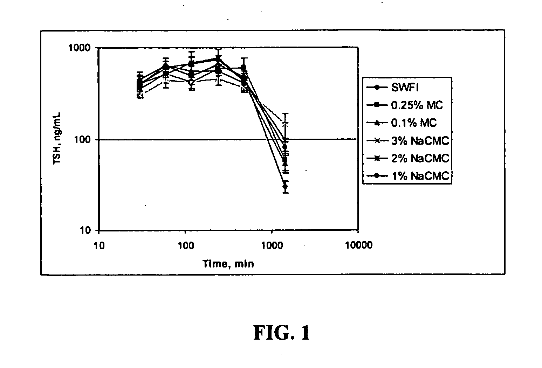

FIG. 1 is a graph depicting the mean serum concentration (ng/mL) of TSH at particular timepoints after intramuscular administration (IM) of different formulations of rhTSH in rats. For each rat, a single dose of 1 mg/kg of rhTSH was administered either in sterile water for injection (labeled as "SWFI"); 0.25% methylcellulose (labeled as "0.25%MC"); 0.1% methylcellulose (labeled as "0.1 %MC"); 3% sodium carboxymethylcellulose (labeled as "3% NaCMC"); 2% sodium carboxymethylcellulose (labeled as "2% NaCMC"); or 1% sodium carboxymethylcellulose (labeled as "1% NaCMC"). Values represent mean ± SD.

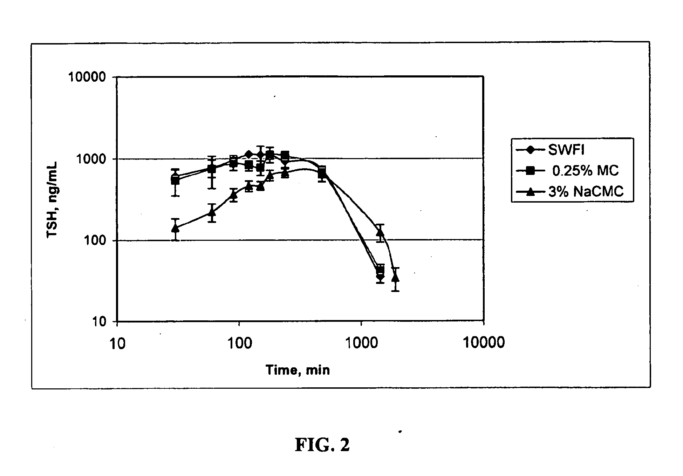

FIG. 2 is a graph depicting the mean serum concentration (ng/mL) of TSH at particular timepoints after intramuscular administration (IM) of different formulations of rhTSH in rats. For each rat, a single dose of 1 mg/kg of rhTSH was administered either in sterile water for injection (labeled as "SWFI"); 0.25% methylcellulose (labeled as "0.25%MC"); or 3% sodium carboxymethylcellulose (labeled as "3% NaCMC").

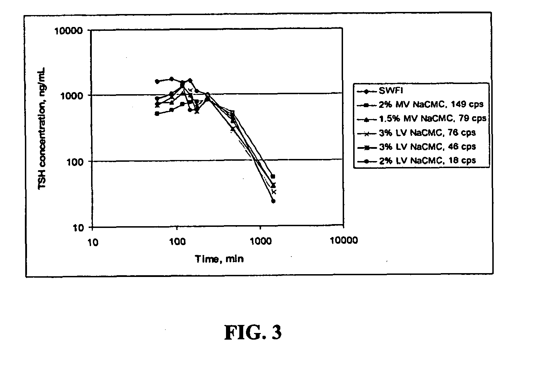

FIG. 3 is a graph depicting the mean serum concentration (ng/mL) of TSH at particular timepoints after intramuscular administration (IM) of different formulations of rhTSH in rats. For each rat, a single dose of 1 mg/kg of rhTSH was administered either in sterile water for injection (labeled as "SWFI"); 2% medium viscosity sodium carboxymethylcellulose from Hercules (labeled as "2% MV NaCMC, 149 cps"); 1.5% medium viscosity sodium carboxymethylcellulose from Hercules (labeled as "1.5% MV NaCMC, 79 cps"); 3% low viscosity sodium carboxymethylcellulose from Ruger (labeled as "3% LV NaCMC, 76 cps"); 3% low viscosity sodium carboxymethylcellulose from Hercules (labeled as "3% LV NaCMC, 46 cps"); or 2% low viscosity sodium carboxymethylcellulose (labeled as "2% LV NaCMC, 18 cps").

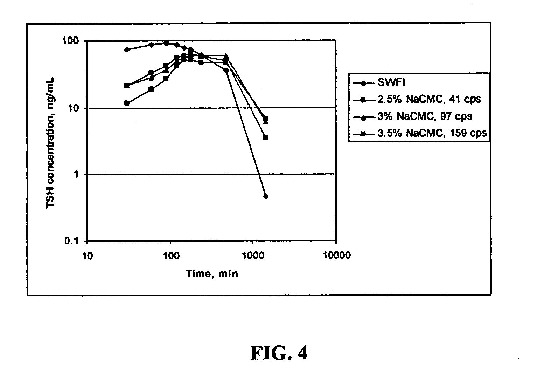

FIG. 4 is a graph depicting the mean serum concentration (ng/mL) of TSH at particular timepoints after intramuscular administration (IM) of different formulations of rhTSH in rats. For each rat, a single dose of 0.1 mg/kg of rhTSH was administered either in sterile water for injection (labeled as "SWFI"); 2.5% sodium carboxymethylcellulose (labeled as "2.5% NaCMC, 41 cps"); 3% sodium carboxymethylcellulose (labeled as "3.0% NaCMC, 97 cps"); or 3.5% sodium carboxymethylcellulose (labeled as "3.5% NaCMC, 159 cps"). Values represent mean ± SD.

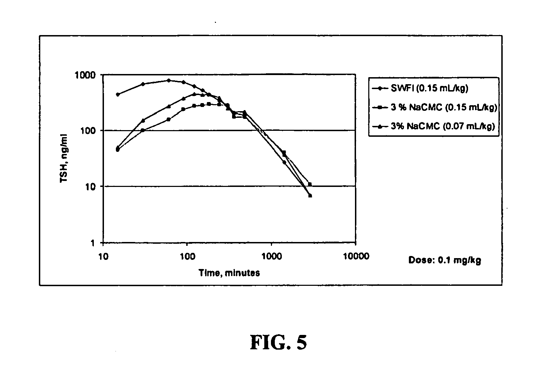

FIG. 5 is a graph depicting the mean serum concentration (ng/mL) of TSH at particular timepoints after intramuscular administration (IM) of different formulations and/or doses of rhTSH in dogs. For each dog, a single dose of 1 mg/kg of rhTSH was administered. Dogs were administered either 0.15 ml/kg rhTSH in sterile water for injection (labeled as "SWFI (0.15 mL/kg)"); 0.15 ml/kg of rhTSH in 3% sodium carboxymethylcellulose (labeled as "3% NaCMC (0.15 mL/kg)"); or 0.07 ml/kg of rhTSH in 3% sodium carboxymethylcellulose (labeled as "3% NaCMC (0.07 mL/kg)").

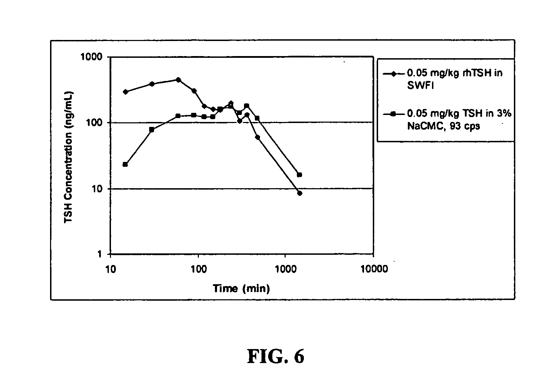

FIG. 6 is a graph depicting the mean serum concentration (ng/mL) of TSH at particular timepoints after intramuscular administration (IM) of different formulations of rhTSH in dogs. Dogs were administered 0.05 mg/kg of rhTSH in either sterile water for injection (labeled as "0.05 mg/kg rhTSH in SWFI") or 3% sodium carboxymethylcellulose at 93 cps (labeled as "0.05 mg/kg rhTSH in 3% NaCMC, 93 cps").

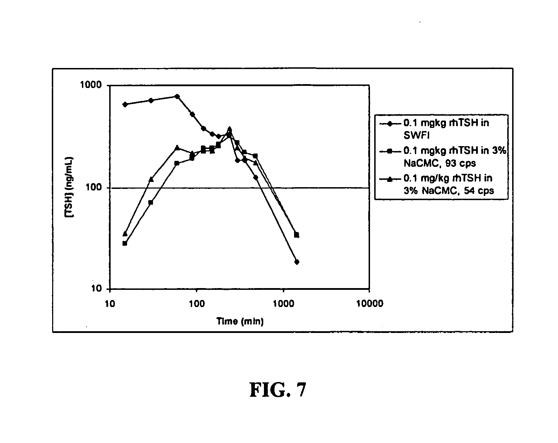

FIG. 7 is a graph depicting the mean serum concentration (ng/mL) of TSH at particular timepoints after intramuscular administration (IM) of different formulations of rhTSH in dogs. Dogs were administered 0.1 mg/kg of rhTSH in sterile water for injection (labeled as "0.1 mg/kg rhTSH in SWFI"); 3% sodium carboxymethylcellulose at 93 cps (labeled as "0.1 mg/kg rhTSH in 3% NaCMC, 93 cps"); or 3% sodium carboxymethylcellulose at 54 cps (labeled as "0.1 mg/kg rhTSH in 3% NaCMC, 54 cps").

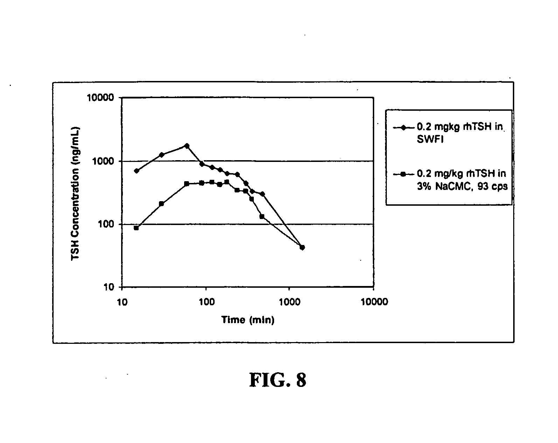

FIG. 8 is a graph depicting the mean serum concentration (ng/mL) of TSH at particular timepoints after intramuscular administration (IM) of different formulations of rhTSH in dogs. Dogs were administered 0.2 mg/kg of rhTSH in either sterile water for injection (labeled as "0.2 mg/kg rhTSH in SWFI") or 3% sodium carboxymethylcellulose at 93 cps (labeled as "0.2 mg/kg rhTSH in 3% NaCMC, 93 cps").

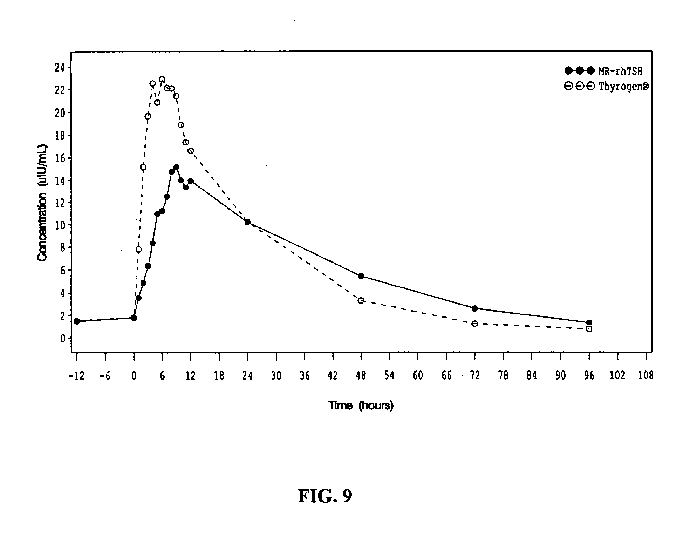

FIG. 9 is a graph depicting the mean uncorrected serum concentration (ng/mL) of TSH at particular timepoints (pre-dose to 96 hours) after intramuscular administration (IM) of 0.1 mg of rhTSH (Thyrogen^{®}) or 0.1 mg of rhTSH in 3% carboxymethylcellulose (labeled as "MR-rhTSH") in humans.

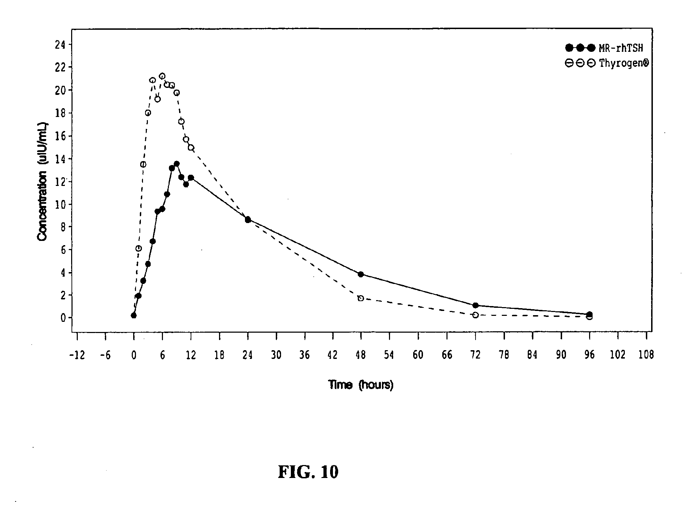

FIG. 10 is a graph depicting the mean corrected serum concentration (ng/mL) of TSH at particular timepoints (pre-dose to 96 hours) after intramuscular administration (IM) of 0.1 mg of rhTSH (Thyrogen^{®}) or 0.1 mg of rhTSH in 3% carboxymethylcellulose (labeled as "MR-rhTSH") in humans.

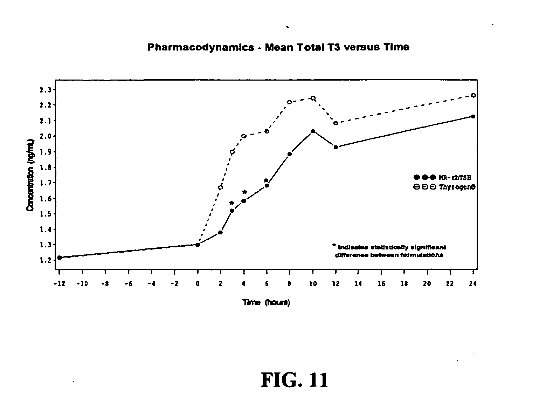

FIG. 11 is a graph depicting the concentration of T₃ (triiodothyronine) in human subjects after intramuscular administration (IM) of 0.1 mg of rhTSH (Thyrogen^{®}) or 0.1 mg of rhTSH in 3% carboxymethylcellulose (labeled as "MR-rhTSH"). * indicates a statistically significant (p<0.05) difference between the treatment groups using T Tests, adjusted for multiple testing using the Bootstrap method.

### DETAILED DESCRIPTION OF THE INVENTION

Throughout this disclosure, various publications, patents and published patent specifications are referenced by an identifying citation. The disclosures of these publications, patents and published patent specifications are intended to more fully describe the state of the art to which this invention pertains.

As used herein, certain terms have the following defined meanings.

### Definitions

As used herein, the term "comprising" is intended to mean that the compositions and methods include the recited elements, but not excluding others. "Consisting essentially of", when used to define compositions and methods, shall mean excluding other elements of any essential significance to the combination. Thus, a composition consisting essentially of the elements as defined herein would not exclude trace contaminants from the isolation and purification method and pharmaceutically-acceptable carriers, such as phosphate-buffered saline, preservatives, and the like. "Consisting of" shall mean excluding more than trace elements of other ingredients and substantial method steps for administering the compositions of this invention. Embodiments defined by each of these transition terms are within the scope of this invention.

All numerical designations, e.g., pH, temperature, time, concentration, and molecular weight, including ranges, are approximations which are varied (+) or (-) by increments of 0.1. It is to be understood, although not always explicitly stated, that all numerical designations are preceded by the term "about". It also is to be understood, although not always explicitly stated, that the reagents described herein are merely exemplary and that equivalents of such are known in the art.

The term "isolated" means separated from constituents, cellular and otherwise, in which the polynucleotide, peptide, polypeptide, protein, antibody, or fragments thereof, are normally associated with in nature. As is apparent to those of skill in the art, a non-naturally-occurring polynucleotide, peptide, polypeptide, protein, antibody, or fragments thereof, does not require "isolation" to distinguish it from its naturally-occurring counterpart. In addition, a "concentrated", "separated" or "diluted" polynucleotide, peptide, polypeptide, protein, antibody, or fragments thereof, is distinguishable from its naturally-occurring counterpart in that the concentration or number of molecules per volume is greater than ("concentrated") or less than ("separated") that of its naturally-occurring counterpart. A polynucleotide, peptide, polypeptide, protein, antibody, or fragments thereof, which differs from the naturally-occurring counterpart in its primary sequence or, for example, by its glycosylation pattern, need not be present in its isolated form since it is distinguishable from its naturally-occurring counterpart by its primary sequence, or alternatively, by another characteristic, such as glycosylation pattern. Thus, a non-naturally-occurring polynucleotide is provided as a separate embodiment from the isolated naturally-occurring polynucleotide. A protein produced in a bacterial cell is provided as a separate embodiment from the naturally-occurring protein isolated from a eukaryotic cell in which it is produced in nature.

The term "recombinant" refers to a polynucleotide synthesized or otherwise manipulated in vitro (e.g., "recombinant polynucleotide"), and to methods of using recombinant polynucleotides to produce gene products in cells or other biological systems, and to a polypeptide ("recombinant protein") encoded by a recombinant polynucleotide.

A "pharmaceutical composition" is intended to include the combination of an active agent with a carrier, inert or active, making the composition suitable for diagnostic or therapeutic use *in vitro, in vivo* or *ex vivo.*

As used herein, the term "pharmaceutically-acceptable carrier" encompasses any of the standard pharmaceutical carriers, such as a phosphate-buffered saline solution, water, and emulsions, such as an oil/water or water/oil emulsion, and various types of wetting agents. The compositions also can include stabilizers and preservatives. For examples of carriers, stabilizers and adjuvants, see Martin, REMINGTON'S PHARM. SCI., 15th Ed. (Mack Publ. Co., Easton (1975)).

An "excipient" refers to an inert substance added to a pharmaceutical composition to further facilitate administration of the active ingredient.

As used herein, the term "a viscous vehicle" intends an inert substance used to dilute, where the substance has a relatively high resistance to flow. It is known that Newtonian liquids (e.g., water) flow as soon as force is applied. Viscosity remains constant regardless of the rate of shear. Viscosity refers to the measure of the internal friction of a fluid that provides resistance to shear (motion) when a fluid is stirred or poured. The greater the forces of internal friction (i.e. the greater the viscosity), the less easily the fluid will flow.

Viscosity is most commonly measured with rotating cylinder viscometers or capillary tube devices. Rotating cylinder viscometers measure the shear forces associated with a fluid, and thus determine the absolute viscosity. The absolute viscosity is usually determined in metric units, which are centipoise. The density of the fluid must then be measured so that the kinematic viscosity may be obtained. Kinematic viscosity is expressed in centistokes in the metric system. When absolute viscosity is expressed in centipoise and density is expressed in gram/cc, the ratio will result in centistokes. The viscosity of a liquid is highly temperature dependent. An increase in temperature will cause a decrease in viscosity. The viscosity measurements herein are considered at room temperature (e.g., 20-25°C).

As used herein, the term "pharmaceutically-acceptable polymer" refers to a polymer that provides a viscous vehicle for the pharmaceutically-acceptable administration of TSH *in vitro, in vivo* or *ex vivo.* In the context of the invention, the polymer is a cellulose derivative.

As used herein, the term "modified-release formulation" refers to a solution of TSH in a viscous vehicle and/or a preparation of TSH that provides a delayed Tₘₐₓ and/or decreased Cₘₐₓ as compared to a corresponding aqueous solution of TSH.

As used herein, the term "modified-release rhTSH" refers to a solution of recombinant human TSH (rhTSH) in a viscous vehicle and/or a preparation of rhTSH that provides a delayed Tₘₐₓ and/or decreased Cₘₐₓ as compared to a corresponding aqueous solution of rhTSH.

As used herein, the term "corresponding aqueous solution of TSH" refers to a solution of TSH in water or buffer that is able to dissolve TSH. A corresponding aqueous solution of TSH is substantially free of a viscous vehicle.

As used herein, the term "metabolically clearable" refers to removal of a compound from a body through normal processes of the body or metabolism.

An "effective amount" is an amount sufficient to effect beneficial or desired results. An effective amount can be administered in one or more administrations, applications, or dosages.

A "subject," "individual" or "patient" is used interchangeably herein, and refers to a vertebrate, preferably a mammal, more preferably a human. Mammals include, but are not limited to, murines, rats, simians, humans, farm animals, sport animals, and pets.

The pharmacokinetic profile of a drug can be determined by a blood (or serum or plasma) concentration time curve, see Ansel et al., PHARMACEUTICAL DOSAGE FORMS AND DRUG DELIVERY SYSTEMS, 7th Ed. (Lippencock, Williams, and Wilkens, Philadelphia, PA (1999)). A blood (or serum or plasma) concentration time curve follows the concentration of a drug over time with a vertical axis depicting a percent of dose and a horizontal axis depicting time. Ansel et al. (1999, supra at page 113) reports that "[w]hen a drug is first administered (time zero), the blood concentration of the drug should also be zero. As the drug passes into the stomach and/or intestine, it is released from the dosage form, eventually dissolves, and is absorbed. As the sampling and analyses continues, the blood samples reveal increasing concentrations of drug until the maximum (peak) concentration (Cₘₐₓ) is reached. Then, the blood level of the drug progressively decreases and, if no additional dose is given, eventually falls to zero." Though Ansel discloses release of a drug into the stomach and/or intestine, it is understood that the release of drug can occur in other locations as well, depending on the route of administration.

In considering parameters for comparative evaluation of blood level curves, Chodos and Santo (BASICS OF BIOAVAILABILITY, Kalamazoo, MI, The Upjohn Company (1973)) listed the following:
1. The Peak Height Concentration (Cₘₐₓ);
2. The Time of the Peak Height Concentration (Tₘₐₓ); and
3. The Area Under the Blood (or serum or plasma) Concentration-Time Curve (AUC).

In this regard, Ansel et al. (1999, supra at page 115) also reports that "changes in the rate of drug absorption will result in changes in the values of both Cₘₐₓ and Tₘₐₓ. Each product has its own characteristic rate of absorption. When the rate of absorption is decreased, the Cₘₐₓ is lowered and Tₘₐₓ occurs at a later time."

An "effective Tₘₐₓ" as used herein refers to a "Time of the Peak Height Concentration", which is characteristic of the composition in reference. An "effective Cₘₐₓ" as used herein refers to a "Peak Height Concentration", which is characteristic of the composition in reference. In many situations, an effective Tₘₐₓ and Cₘₐₓ provide a blood (or serum or plasma) concentration time curve in which the concentration of a drug is in a therapeutic range.

Administration of TSH is a diagnostic and/or therapeutic approach for thyroid conditions, such as goiter and thyroid cancer. Administration of TSH to a patient can alter the metabolism of the goiter tissue, the normal thyroid tissue, and/or the thyroid cancer tissue. The change in tissue metabolism can allow diagnostic and/or therapeutic procedures for these thyroid diseases to be conducted, which sometimes make use of radioactive isotopes, such as, but not limited to, radioiodine (123-I, 124-I, 131-I), radioactively-labeled glucose (such as used for PET scanning), as well as nonradioactive strategies (such as stimulating tissue metabolism by TSH and then measuring a tumor marker in blood, such as thyroglobulin).

The pharmacokinetics of the appearance of TSH in the bloodstream can affect the effectiveness of the diagnostic and/or therapeutic procedure(s). Thus, use of a formulation(s) to tailor the pharmacokinetics of TSH is an important strategy for optimizing the diagnostic and/or therapeutic approach to patients with goiter, thyroid cancer or other thyroid disease(s). In the application of this principle to goiter, for example, a more gradual release of TSH into the bloodstream over a day or two is desired because a sudden spike of TSH serum level can result in hyperthyroidism in a patient. For certain situations (for example, goiter), a gradual release of TSH into the bloodstream over several hours, such as 1, 2, 3, 4, 5, 6, 7, 8, 9, 10, 11, 12, 13, 14, 15, 16, 17, 18, 19, 20, 25, 30, 35, 40, 45 hours or perhaps as long as 50 hours, may be useful. Alternatively, for certain situations, such as thyroid cancer, a gradual release of TSH into the bloodstream over several days, such as 1, 2, 3, 4, 5, 10, 15, 20, 25 days or perhaps as long as 30 days, may be useful for stimulating the metabolism of thyroid cancer tissue that remains in the body so that it can be detected and/or located by use of tumor markers or nuclear medicine or other scanning, and this stimulation of the tumor may also make the cancer more treatable using radiation or other means. Thus, a need exists for TSH-containing formulations that optimize the pharmacokinetics of TSH release.

Administration of TSH affects the release of T₃ (triiodothyronine), which is a thyroid hormone that circulates in the blood, mostly bound (about 99.5%) to carrier proteins. Unbound T₃ is believed to be responsible for biological action and control of metabolism. In normal thyroid function, the concentration of carrier proteins can change, and the total T₃ level can change, but the level of free T₃ remains constant. The constancy of T₃ levels may not be the case for an abnormally-functioning thyroid. Therefore, the level of free T₃ can correlate with a clinical status. A spike in free T₃ level can result in hyperthyroidism symptoms and/or cardiac symptoms. For example, spikes in free T₃ levels can result in increased heart rate, which can be a concern for patients (e.g., older patients, sicker patients). Thus, in the administration of TSH, a sudden spike of serum TSH level can result in a sudden spike in free T₃ level, which can, in turn, result in hyperthyroidism and/or cardiac symptoms in a patient. Therefore, in particular embodiments, it is desirable to administer TSH such that free T₃ levels are maintained at a more constant level.

In one embodiment, Applicants' disclosure provides a method for providing a modified-release formulation for the administration of TSH by admixing an effective amount of TSH and an effective amount of a viscous vehicle. Increasing the viscosity of the medium in which TSH is formulated can significantly decrease the rate of absorption of TSH into the tissues surrounding the injection site, which in turn produces a more gradual increase in serum TSH levels. A more gradual release of serum TSH level can result in less of an immediate release of free T₄ (thyroxine), T₄, T₃ and/or free T₃, and cause fewer acute signs and symptoms of hyperthyroidism.

Pharmaceutically-acceptable polymers can increase viscosity without adverse side effects. Accordingly, pharmaceutically-acceptable polymers were used and shown to be successful in altering the pharmacokinetic profile of TSH, when compared to an aqueous solution of TSH. Examples of suitable pharmaceutically-acceptable cellulose derivatives are methylcellulose, hydroxymethylcellulose, hydroxyethylcellulose, hydroxypropyl cellulose, hydroxypropyl methylcellulose, carboxymethylcellulose, ethyl hydroxyethyl cellulose, hypromellose, and calcium carboxymethyl cellulose, -or salts or esters thereof. In one embodiment, the pharmaceutically-acceptable polymer is selected from the group consisting of methylcellulose and carboxymethylcellulose. In another embodiment, the pharmaceutically-acceptable polymer is carboxymethylcellulose.

The disclosure provides for a composition comprising a pharmaceutically-acceptable polymer that is able to alter the pharmacokinetic profile of TSH, when compared to an aqueous solution of TSH. In one embodiment, there is provided a pharmaceutical dosage form comprising TSH and a pharmaceutically-acceptable polymer that allows modified release of the TSH into a bloodstream of a patient, wherein, when administered to the patient, the pharmaceutical dosage form provides an effective Tₘₐₓ of TSH in the patient's serum that is at least about 20% longer than the effective Tₘₐₓ of TSH in the patient's serum when a corresponding aqueous solution of TSH is administered. In another embodiment, there is provided a pharmaceutical dosage form comprising TSH and a pharmaceutically-acceptable polymer that allows modified release of the TSH into a bloodstream of a patient, wherein, when administered to the patient, the pharmaceutical dosage form provides an effective Cₘₐₓ of TSH in the patient's serum that is at least about 20% lower than the effective Cₘₐₓ of TSH in the patient's when a corresponding aqueous solution of TSH is administered.

In one embodiment, Applicants' disclosure provides a method for providing a modified-release formulation for the administration of TSH by admixing an effective amount of TSH and an effective amount of a pharmaceutically-acceptable polymer. In another embodiment, Applicants' disclosure provides a method for providing a modified-release formulation for the administration of TSH by admixing an effective amount of TSH and an effective amount of methylcellulose or carboxymethylcellulose. Other pharmaceutically-acceptable polymers may be identified by admixing various concentrations of polymer and TSH as described herein. Polymers having the same or similar Cₘₐₓ and/or Tₘₐₓ are then identified and can be utilized in the methods described herein. For example, FIG. 2 compares the release kinetics of compositions comprising 1) TSH and water; 2) TSH and methylcellulose; and 3) TSH and carboxymethylcellulose.

The effect of viscosity of a polymer, methylcellulose, on the release rate of chloroquine was reported in Prakongpan et al. (1989) "An improved formulation of chloroquine for intramuscular administration: absorption kinetics in rabbits" J. Pharm. Pharmacol., 41:726-728. As reported by the authors, chloroquine has unusual pharmacokinetic properties, including a central apparent volume of distribution that is several orders of magnitude smaller than the total distribution volume and rapid absorption after intramuscular or subcutaneous injection. Id. Consequently, transiently high blood concentrations of chloroquine can cause a fall in blood pressure that can be lethal to vulnerable subjects. Id. Here, the authors reported a linear relationship between log release rate and log viscosity of the polymer methylcellulose (2%), which was used as the carrier.

Applicants have unexpectedly discovered that combination of TSH and a pharmaceutically-acceptable polymer provides a composition having a modified pharmacokinetic profile that is suitable for the treatment of thyroid conditions. As described and exemplified herein, the formulation unexpectedly provides decreased Cmax and increased Tmax without altering bioavailability of the drug.

To obtain the composition, between about 10 µg to about 5000 µg of TSH is admixed with about 0.2 % to about 5.0 % w/v solution of a pharmaceutically-acceptable polymer. In one embodiment, between about 300 µg/ml to about 1500 µg/ml of TSH is admixed with a pharmaceutically-acceptable polymer. In other embodiments, the amount of pharmaceutically-acceptable polymer is between about 0.25% and about 5.0%; between about 1% and about 4.5%; between about 2% and about 4.0%; between about 2.5% and about 3.5%; or alternatively about 0.2%; about 0.25%; about 0.3%; about 0.35%; about 0.4%; about 0.45%; about 0.5%, about 1%, about 2%, about 3%, or about 4%, of the polymer.

TSH for the formulation can be purified from naturally-occurring mammalian sources, such as bovine, porcine, primate, or human, or alternatively isolated from non-naturally-occurring sources using methods known in the art, such as described in U.S. Patent Nos. 5,840,566 and 6,365,127. Human recombinantly-produced TSH has been utilized in the examples provided below but the inventions of this disclosure should not be limited to the sources provided in the examples. Moreover, as is apparent to those of skill in the art, minor modifications to the protein can be made without departing from the spirit and scope of this invention as long as the composition provides the pharmacokinetic profile within the parameters set forth herein.

Thyrogen^{®} (Genzyme Corp., NDA 2-898) is recombinant human TSH (rhTSH) currently marketed for the diagnosis and/or treatment of thyroid cancer. It is sold as a lyophilized powder for reconstitution with water prior to intramuscular administration. Given that Thyrogen^{®} drug product is stable for 36 months when stored at 2 to 8 °C, it is suitable as a source of TSH for the current formulations.

To increase the viscosity of the medium, a viscous vehicle is used. A pharmaceutically-acceptable polymer can act as a viscous vehicle. The viscous vehicle and its concentration is selected to achieve a formulation having a resultant viscosity of at least about 5 cps, at least about 10 cps; at least about 15 cps; at least about 20 cps; at least about 25 cps; at least about 30 cps; at least about 35 cps; at least about 40 cps; at least about 45 cps; at least about 50 cps; at least about 55 cps; at least about 60 cps; at least about 65 cps; at least about 70 cps; at least about 75 cps; at least about 80 cps; at least about 85 cps; at least about 90 cps; at least about 95 cps; at least about 100 cps; at least about 105 cps; at least about 110 cps; at least about 115 cps; at least about 120 cps; at least about 125 cps; at least about 130 cps; at least about 135 cps; at least about 140 cps; at least about 145 cps; at least about 150 cps; at least about 155 cps; at least about 160 cps; at least about 165 cps; at least about 170 cps; at least about 175 cps; at least about 180 cps; at least about 185 cps; at least about 190 cps; at least about 195 cps; at least about 200 cps; or alternatively between about 40 and 90 cps. In certain embodiments, the viscous vehicle and its concentration is selected to achieve a resultant viscosity of between about 15 and 150 cps, about 40 and 160 cps, about 45 and 125 cps, or about 40 to 90 cps. In a particular embodiment, the viscous vehicle and its concentration is selected to achieve a resultant viscosity of between about 45 and 125 cps.

This disclosure also provides in several embodiments compositions or formulations exhibiting modified-release pharmacokinetic profiles.

One embodiment provides a pharmaceutical dosage form comprising TSH and a pharmaceutically-acceptable polymer that allows modified release of the TSH into the serum of a patient. In one embodiment, the pharmaceutically-acceptable polymer is a viscous vehicle or a pharmaceutically-acceptable diluent as defined herein.

In one embodiment, the compositions contain TSH in a pharmaceutically-acceptable polymer. In one embodiment, the pharmaceutically-acceptable polymer is sodium carboxymethylcellulose. In one embodiment, the composition contains from between about 10 µg/ml to about 5000 µg/ml of TSH in the pharmaceutically-acceptable polymer. In other embodiments, the composition contains from between about 10 µg/ml to about 1500 µg/ml, from between about 10 µg/ml to about 1000 µg/ml, from between about 10 µg/ml to about 800 µg/ml, from between about 10 µg/ml to about 500 µg/ml, from between about 10 µg/ml to about 300 µg/ml, from between about 10 µg/ml to about 200 µg/ml, from between about 10 µg/ml to about 100 µg/ml, or from between about 10 µg/ml to about 90 µg/ml of TSH in the pharmaceutically-acceptable polymer. In one embodiment, the composition contains from between about 40 µg/ml to about 80 µg/ml of TSH in the pharmaceutically-acceptable carrier. In other embodiments, the composition contains about 30 µg/ml, about 40 µg/ml, about 50 µg/ml, about 60 µg/ml, about 70 µg/ml, about 80 µg/ml, or about 90 µg/ml of TSH in the pharmaceutically-acceptable carrier. In a particular embodiment, the composition contains about 60 µg/ml of TSH in the pharmaceutically-acceptable carrier.

The compositions may optionally contain other agents, such as preservatives and the like. In a further embodiment, the compositions are packaged individually for combination by the treating physician or patient. The package optionally may contain a means to administer the composition, such as a syringe and needle. Thus, this disclosure also provides an article of manufacture containing a source of TSH, a source of a suitable pharmaceutically-acceptable polymer, instructions and a means to administer the composition after it has been reconstituted. Still further, the composition is pre-mixed and sold with the means for administration, with or without instructions for its administration.

One embodiment provides a method for providing a modified-release formulation for the administration of TSH by admixing an effective amount of TSH and an effective amount of a pharmaceutically-acceptable polymer.

In one embodiment, the TSH is TSH isolated from a mammal (e.g., a human). In another embodiment, the TSH is recombinant mammalian TSH (e.g., recombinant human TSH).

In one embodiment, the pharmaceutically-acceptable polymer is a metabolically clearable polymer. In another embodiment, the pharmaceutically-acceptable polymer is injectable in a body.

In one embodiment, the dosage form has a viscosity of at least about 20 cps. In another embodiment, the dosage form has a viscosity of at least about 40 cps. In certain embodiments, the composition has a viscosity of at least about 50 cps, at least about 70 cps or at least about 90 cps. In certain embodiments, the dosage form has a viscosity of between about 15 and 150 cps, about 40 and 160 cps, about 45 and 125 cps, or about 40 to 90 cps. In a particular embodiment, the dosage form has a viscosity of between about 45 and 125 cps.

In one embodiment, the pharmaceutically-acceptable polymer comprises sodium carboxymethylcellulose having an average molecular weight between about 90,000 and about 700,000. In another embodiment, the pharmaceutically-acceptable polymer comprises about 0.05 to about 5% sodium carboxymethylcellulose.

As described herein, the formulations and compositions of the invention comprise pharmaceutically-acceptable polymers . In one particular example, the pharmaceutically-acceptable polymer is sodium carboxymethylcellulose having an average molecular weight selected from the group consisting of: about 90,000; about 250,000; and about 700,000. Other suitable polymers (e.g., those described herein) have molecular weights in the range of about 70,000, about 90,000, about 100,000, about 150,000, about 200,000, about 250,000, about 300,000, about 350,000, about 400,000, about 450,000, about 500,000, about 550,000, about 600,000, about 650,000, about 700,000, about 750,000, about 800,000, about 850,000, about 900,000, or about 950,000. Yet further, polymers having molecular weights in the range of between about 70,000 and about 950,000 can be used, or yet further between about 90,000 and about 900,000.

The compositions may optionally contain other agents, such as preservatives and the like. In a further embodiment, the compositions are packaged individually for combination by the treating physician or patient. The package optionally may contain a means to administer the composition, such as a syringe and needle. Thus, this disclosure also provides an article of manufacture containing a source of TSH, a source of a suitable pharmaceutically-acceptable polymer, instructions and a means to administer the composition after it has been reconstituted. Still further, the composition is pre-mixed and sold with the means for administration, with or without instructions for its administration.

One embodiment provides a method for providing a modified-release formulation for the administration of TSH by admixing an effective amount of TSH and an effective amount of a pharmaceutically-acceptable polymer.

In one embodiment, the TSH is TSH isolated from a mammal (e.g., a human). In another embodiment, the TSH is recombinant mammalian TSH (e.g., recombinant human TSH).

In one embodiment, the pharmaceutically-acceptable polymer is a metabolically-clearable polymer. In another embodiment, the pharmaceutically-acceptable polymer is injectable in a body.

Another embodiment provides a pharmaceutical dosage form comprising TSH and a pharmaceutically-acceptable polymer that allows modified release of the TSH into a bloodstream of a patient, wherein, when administered to the patient, the pharmaceutical dosage form provides an effective Cₘₐₓ of TSH in the patent's serum that is at least about 20% longer than the effective Tₘₐₓ of TSH in the patient's serum when a corresponding aqueous solution of TSH is administered.

In one embodiment, the pharmaceutical composition comprises an effective amount of TSH and an effective amount of a pharmaceutically-acceptable polymer, wherein the composition provides an effective Tₘₐₓ of at least six hours after administration to a patient in need thereof.

One embodiment provides a pharmaceutical dosage form comprising TSH and a pharmaceutically-acceptable polymer that allows modified release of the TSH into a bloodstream of a patient, wherein, when administered to the patient, the pharmaceutical dosage form provides an effective Cₘₐₓ of TSH in a serum of the patient at least about 20% lower than an effective Cₘₐₓ of TSH in the serum of the patient when a corresponding aqueous solution of TSH is administered.

Another embodiment provides a pharmaceutical composition comprising an effective amount of TSH and an effective amount of a pharmaceutically-acceptable polymer, wherein the composition provides an effective serum Cₘₐₓ of greater than about 2.0 mIU/L to a patient in need thereof.

One embodiment provides a pharmaceutical composition comprising an effective amount of TSH and an effective amount of a pharmaceutically-acceptable polymer, wherein the composition provides a serum T₃ level in patient in need thereof of no more than 2.5 ng/ml over a 48-hour period after administration.

In another embodiment, the patient in need thereof is a patient suffering from a thyroid condition selected from goiter and thyroid cancer.

As is apparent to the skilled artisan, one or more suitable pharmaceutically-acceptable diluents can be combined for use in preparing a formulation as described herein. The compositions may optionally contain other agents, such as preservatives and the like. In a further embodiment, the compositions are packaged individually for combination by the treating physician or patient. The package optionally may contain a means to administer the composition, such as a syringe and needle. Thus, this disclosure also provides an article of manufacture containing a source of TSH, a source of a suitable diluent, instructions and a means to administer the composition after it has been reconstituted. Still further, the composition is pre-mixed and sold with the means for administration, with or without instructions for its administration.

The compositions of this invention, when administered to a patient in need thereof (e.g., a thyroid cancer patient, a patient suffering from goiter, a patient suffering from another thyroid condition(s)), will provide a blood serum concentration of TSH that has been tailored for the indicated use. In one embodiment, the composition provides a serum T₃ level in a patient in need thereof of no more than 2.5 ng/ml over a 48-hour period. In another embodiment, the composition provides an effective Tₘₐₓ of at least six hours after administration to a patient in need thereof. In another embodiment, the composition provides an effective Cₘₐₓ of greater than about 2.0 mIU/L to a patient in need thereof.

In one embodiment, the thyroid condition to be treated is goiter and the serum Cₘₐₓ of TSH should exceed about 2.0 mIU/L starting one hour after administration. In another embodiment, the serum Cₘₐₓ of TSH exceeds about 5 mIU/L starting one hour after administration. In still another embodiment, the thyroid condition to be treated is goiter and the formulation is adjusted so that the TSH serum Cₘₐₓ occurs about a few hours (e.g., about 8 to 12 hours) after administration to best optimize thyroid uptake of radioiodine without causing acute release of thyroid hormones. For certain indications (e.g., goiter), a gradual release of TSH into the bloodstream over several hours, such as 1, 2, 3, 4, 5, 6, 7, 8, 9, 10, 11, 12, 13, 14, 15, 16, 17, 18, 19, 20, 25, 30, 35, 40, 45 hours and perhaps for up to 50 hours may be useful.

For other indications (e.g., thyroid cancer), a different optimal pharmacokinetics of serum TSH may hold for the diagnostic and/or therapeutic application being attempted, such as achieving a slow release of TSH into the bloodstream over several days, such as 1, 2, 3, 4, 5, 10, 15, 20, 25 days and perhaps up to 30 days. The formulations can be delivered by any suitable method, such as intramuscular injection.

In particular embodiments, the invention is the use of the compositions of the invention to treat a thyroid condition. In other embodiments, the invention is the use of the compositions of the invention for the manufacture of a medicament for treating a thyroid condition. In many instances, the thyroid condition results from an altered level of TSH. Levels of TSH can be monitored and analyzed with a blood test measuring the amount of TSH, where normal serum levels of TSH are generally between about 0.4 to about 4.5 mIU/L in adults and about 3 to about 20 mIU/L in newborns. Hence, an altered level of TSH is a level outside normal levels of TSH. Conditions resulting from an altered level of TSH include goiter and hypothyroidism. A positive diagnosis of hypothyroidism can be made with a blood test showing elevated levels of TSH, where normal levels of TSH are generally between about 0.4 to about 4.5 mIU/L in adults and about 3 to about 20 mIU/L in newborns. A positive diagnosis of goiter can be ascertained from a blood test showing altered levels of TSH. In a particular embodiment, the thyroid condition is selected from the group consisting of goiter and thyroid cancer.

In another embodiment, the invention is a method for maintaining blood plasma concentration of TSH above 2.0 mIU/L in a patient suffering from a thyroid condition, comprising administering to the patient an effective amount of a disclosed pharmaceutical composition, wherein the blood plasma concentration of TSH is maintained for about one hour after administration For certain indications (e.g., goiter), a gradual release of TSH into the bloodstream over several hours, such as 1, 2, 3, 4, 5, 6, 7, 8, 9, 10, 11, 12, 13, 14, 15, 16, 17, 18, 19, 20, 25, 30, 35, 40, 45 hours and perhaps for up to 50 hours may be useful. Alternatively, for certain situations, such as for thyroid cancer, a gradual release of TSH into the bloodstream over several days, such as 1, 2, 3, 4, 5, 10, 15, 20, 25 days and perhaps for up to 30 days may be useful.

One embodiment provides a method for treating a thyroid condition in a patient in need thereof, comprising delivering to the patient an effective amount of the pharmaceutical composition comprising an effective amount of TSH and an effective amount of pharmaceutically-acceptable polymer.

In one embodiment, the pharmaceutical composition is delivered via intramuscular injection.

In another embodiment, the pharmaceutical composition delivers an effective Tₘₐₓ of TSH in a serum of the patient is at least about 20% longer compared to an effective Tₘₐₓ of TSH in the serum of the patient when a corresponding aqueous solution of TSH is administered.

In another embodiment, the pharmaceutical composition delivers an effective Cₘₐₓ of TSH in the patient's serum that is at least about 20% lower than the effective Cₘₐₓ of TSH in the patient's serum when a corresponding aqueous solution of TSH is administered.

In another embodiment, the pharmaceutical composition provides a serum T₃ level in patient in need thereof of no more than 2.5 ng/ml over a 48-hour period after administration.

Another embodiment provides a method for maintaining blood plasma concentration of TSH above 2.0 mIU/L in a patient suffering from a thyroid condition, comprising administering to the patient an effective amount of the pharmaceutical composition comprising an effective amount of TSH and an effective amount of pharmaceutically-acceptable polymer, wherein the blood serum or plasma concentration of TSH is maintained above about 2.0 mIU/L for longer than about six hours after administration.

In other embodiments, the blood plasma concentration of TSH remains elevated for longer than about ten hours, longer than about fifteen hours, longer than about one day, longer than about two days or longer than about four days, after administration.

Another embodiment provides a method for treating a thyroid condition in a patient, comprising administering to the patient an effective amount of the pharmaceutical composition of the disclosed embodiments, wherein the composition provides a serum T₃ level in patient in need thereof of no more than 2.5 ng/ml over a 48-hour period after administration.

### EXAMPLES

It is understood that the invention is not limited to the embodiments set forth herein for illustration, but embraces all such forms thereof as come within the scope of the above disclosure.

In the examples below as well as throughout the application, the following abbreviations have the following meanings. If not defined, the terms have their generally accepted meanings.

### Abbreviations

| | |
|---|---|
| rTSH | recombinant TSH |
| | |
| w/w | weight to weight |
| AUC | Area under the curve |
| Cₘₐₓ | Maximum plasma concentration |
| EKG | Electrocardiograms |
| IM | Intramuscular |
| MRrhTSH | Modified-release recombinant thyroid stimulating hormone |
| NaCMC | Sodium carboxymethylcellulose |
| PD | Pharmacodynamics |
| PK | Pharmacokinetics |
| rhTSH | Recombinant human thyroid stimulating hormone |
| Tₘₐₓ | Time to maximum concentration |
| T₃ | Triiodothyronine |
| T₄ | Thyroxine |

### Example 1: Preparation of Solution containing Sodium Carboxymethylcellulose or Methylcellulose

Sodium carboxymethylcellulose and methylcellulose were obtained from Spectrum Pharmaceuticals (Irvine, CA). Solutions of 3% sodium carboxymethylcellulose and 1% sodium carboxymethylcellulose were prepared. A solution of 0.5% methylcellulose was prepared.

A solution of TSH in 3% mannitol, 0.2 % sodium chloride, 20 mM phosphate buffer, pH 7.0 (1 ml of 0.9 mg/ml solution) was added to a solution of 3% sodium carboxymethylcellulose (1 ml) and to a solution of 0.5% methylcellulose (1 ml). The solutions were vortexed and observed against fluorescent lighting with a white and black background. Each of the mixed solutions was effervescent. After sitting for about 5 minutes, each of the solutions was clear and there were no visible particles in the solution. When the solutions were shaken, effervescence appeared, but there were still no visible particles.

A lyophilized cake of TSH was reconstituted with a solution of 3% sodium carboxymethylcellulose (10 ml) and with a solution of 0.5% methylcellulose (10 ml). Each of the reconstituted solutions was not opalescent or milky white. The sodium carboxymethylcellulose solution reconstituted the lyophilized cake of TSH quicker than the methylcellulose solution, but eventually both solutions reconstituted the lyophilized cake of TSH. Effervescence was observed for both solutions. There were no particulates in both solutions.

### Pharmacokinetics of rhTSH administered to rats: Effect of and methylcellulose concentrations

In this Example, the pharmacokinetics (PK) of six different formulations of rhTSH were compared. The study design comprised 18 jugular vein cannulated rats divided into 6 groups. All rats were administered a single dose of 1 mg/kg recombinant human TSH (rhTSH) through intramuscular injection (IM). The 6 administration vehicles were sterile water for injection different percentages of methylcellulose (MC) or sodium carboxymethylcellulose (NaCMC) at approximately 0.9 mg/mL. In particular:

Group 1 was administered rhTSH in sterile water for injection (SWFI);

Group 2 was administered rhTSH in 0.25% MC;

Group 3 was administered rhTSH in 0.1% MC;

Group 4 was administered rhTSH in 3% NaCMC;

Group 5 was administered rhTSH in 2% NaCMC; and

Group 6 was administered rhTSH in 1% NaCMC.

Serum samples were taken for PK analysis (n=3) at 0, 30, 60, 120, 240, 480, and 1440 minutes. Serum samples were evaluated using a rhTSH ELISA.

### Materials and Methods

The rats used in this Example were female Sprague Dawley rats (Charles River Laboratories, Wilmington, MA) weighing approximately 250 g. As indicated, all rats were administered a single dose of 1 mg/kg recombinant human TSH (rhTSH) through intramuscular injection (IM). The 6 test articles were formulated either with sterile water for injection or different percentages of methylcellulose (MC) or sodium carboxymethylcellulose (NaCMC) at approximately 0.9 mg/mL. Specifically, the rats were administered rhTSH in sterile water for injection (SWFI), rhTSH in 0.25% MC, rhTSH in 0.1% MC, rhTSH in 3% NaCMC, rhTSH in 2% NaCMC, or rhTSH in 1% NaCMC.

### rhTSH ELISA.

A colorimetric sandwich enzyme linked immunosorbant assay (ELISA) using mouse anti-hCG antibody and biotinylated mouse anti-rhTSH antibody was employed to quantitate the amount of rhTSH in rat serum. Plates were coated with mouse anti-hCG antibody (Scantibodies Laboratories Inc., Santee, CA) and were incubated overnight at 2 to 8°C. A standard curve was prepared using rhTSH starting at 5.556 ng/ml and serially diluting 1:1.5 to 0.488 ng/ml. A series of dilutions were prepared for each test sample in dilution buffer. Standards, controls, and samples were added to the plates in duplicate and were incubated for one hour at 37°C with shaking. Plates were washed with ELISA plate wash solution (1X Phosphate-Buffered Saline with 0.5% Tween20™ (polyoxyethylene sorbitan monolaurate); Perkin Elmer Life Sciences Products) Biotinylated mouse anti-rhTSH was diluted appropriately in sample diluent buffer, added to each well, and incubated for one hour at 37°C with shaking. Plates were washed six times with ELISA plate wash solution. Streptavidin horseradish peroxidase (Pierce Biotechnology, Inc., Rockford, IL) was diluted appropriately in sample diluent buffer (1X Phosphate-Buffered Saline, 0.5% Tween20™ (polyoxyethylene sorbitan monolaurate), 0.1% BSA; Perkin Elmer Life Sciences Products) was added to each well, and incubated for 15 minutes at room temperature in the dark. Plates were washed with ELISA plate wash solution. Ortho-phenylenediamine (OPD) (Sigma, St. Louis, MO) was added and incubated for 30 minutes at 37°C with shaking. The reaction was stopped using a 4.5M sulfuric acid stop solution (Fisher Scientific, Hampton, NH). The amount of rhTSH in each sample was measured at an absorbance reading of 490 nm.

### Data Analysis

Absorbance readings for samples were excluded if the absorbance measurement at 490 nm was greater than that achieved by the highest rhTSH standard (5.556 ng/ml), or less than the reading obtained for the lowest rhTSH standard (0.488 ng/ml). Such readings were not included in the data analysis.

The amount of rhTSH (measured in ng/ml) in each sample dilution was interpolated from the standard curve. The amount of rhTSH in each sample was calculated by multiplying the appropriate dilution factor by the interpolated result. The amount of rhTSH is expressed as ng of rhTSH/ml of serum. Table 1 provides a summary of the parameters for the study. No abnormalities in the animals were observed during the in-life portion of the study or at necropsy.

**Table 1: Study Design**

| Group # | # of Animals | Dose mg/kg | Conc mg/mL | Dose Route | Test Article | Timepoints |
|---|---|---|---|---|---|---|
| 1 | 3 | 1 | 0.9 | IM | rhTSH in SWFI | Pre-dose, 30 min, 1 hr, 2 hr, 4 hr, 8 hr and 24 hr. |
| 2 | 3 | | | | rhTSH in 0.25% MC | |
| 3 | 3 | | | | rhTSH in 0.1% MC | |
| 4 | 3 | | | | rhTSH in 3 % NaCMC | |
| 5 | 3 | | | | rhTSH in 2 % NaCMC | |
| 6 | 3 | | | | rhTSH in 1% NaCMC | |

### Pharmacokinetic Parameters

Pharmacokinetic analysis was performed on individual animals. Pharmacokinetic analysis of serum concentration-time data was performed using WinNonlin^{®} computer software (Pharsight Corporation, Mountain View, CA). The terminal elimination half-life (t_{1/2}) represents the time required for the drug concentration at any point on the straight line (log-linear scale) to decrease by one-half, and is given by 0.693/n, where n is the elimination rate constant (the product of 2.303 and the terminal slope). The Cₘₐₓ is the maximum serum concentration in ng/mL. The Tₘₐₓ is the time at which maximum concentration was observed (time at Cₘₐₓ) in minutes. The Cₘₐₓ, Tₘₐₓ, terminal elimination half-life (t_{1/2}), area under the curve (AUC) and clearance (Cl) parameters are summarized in Table 2. FIG. 1 depicts the mean serum concentration (ng/mL) of TSH at particular timepoints after intramuscular administration (IM) of different MC or NaCMC formulations of rhTSH in rats.

**Table 2: Cₘₐₓ, Tₘₐₓ, t_{1/2}, AUC and Cl Values for SWFI, MC and NaCMC Formulations**

| | **Cₘₐₓ ng/mL** | **Tₘₐₓ min** | **t_{1/2} min** | **AUC (all) min*ng/mL** | **Cl mL/min/kg** |
|---|---|---|---|---|---|
| rhTSH in SWFI | 661.20 ± 100.26 | 180.0 ± 103.9 | 262.03 ± 21.90 | 504042.63 ± 35351.93 | 1.9 ± 0.1 |
| rhTSH in 0.25% MC | 645.27 ± 134.23 | 320.0 ± 138.6 | 333.77 ± 35.03 * | 569415.57 ± 117258.69 | 1.7 ± 0.3 |
| rhTSH in 0.1 % MC | 662.97 ± 126.45 | 80.0 ± 34.6 | 349.37 ± 24.48 * | 463768.20 ± 66534.84 | 2.0 ± 0.3 |
| rhTSH in 3% NaCMC | 497.23 ± 39.78 * | 120.0 ± 103.9 | 740.83 ± 190.33* | 436426.43 ± 8137.11* | 1.7 ± 0.2 |
| rhTSH in 2 % NaCMC | 811.53 ± 136.68 | 200.0 ± 69.3 | 436.50 ± 160.01 | 548849.13 ± 77824.65 | 1.6 ± 0.2 |
| rhTSH in 1 % NaCMC | 769.33 ± 144.44 | 200.0 ± 69.3 | 390.33 ± 40.07 * | 529652.40 ± 86106.76 | 1.7 ± 0.2 |

| | | | | | |
|---|---|---|---|---|---|
| * P value < 0.05 | | | | | |

### Summary of Pharmacokinetic Parameters

Following the bolus IM administration of each rhTSH preparation, the semilogarithmic plot of concentration versus time demonstrated an absorption, distribution and elimination profile consistent with route of administration. Administration of rhTSH reconstituted in 3% NaCMC resulted in a statistically-significant shift of Cₘₐₓ when compared to rhTSH in SWFI (see, e.g., Table 2 and FIG. 1). There was also an indication of an effect with rhTSH reconstituted in MC. Example 3: Comparison of Sodium Carboxymethylcellulose and Methylcellulose in rats

In this Example, the pharmacokinetics (PK) of three different formulations of rhTSH were compared. The study design consisted of 14 jugular vein cannulated rats divided into 3 groups. All rats were administered a single dose of 1 mg/kg rhTSH through intramuscular injection (IM). The 3 administration vehicles were sterile water for injection (SWFI),0.25 % methylcellulose (MC) and 3% sodium carboxymethylcellulose (NaCMC) at approximately 0.9 mg/mL. In particular:

Group 1 was administered rhTSH in sterile water for injection (SWFI);

Group 2 was administered rhTSH in 0.25% MC; and

Group 3 was administered rhTSH in 3% NaCMC.

Serum samples were taken for PK analysis (n=3) at 0, 30, 60, 90, 120, 150, 180, 240, 480, 1440, and 1920 minutes. Serum samples were evaluated using the rhTSH ELISA.

### Materials and Methods

Experiments were performed as described in Example 2.

Table 3 provides a summary of the parameters for the study. No abnormalities in the animals were observed during the in-life portion of the study or at necropsy.

**Table 3: Study Design**

| Group # | # of Animals | Dose mg/kg | Conc. mg/mL | Dose Route | Test Article | Timepoints |
|---|---|---|---|---|---|---|
| 1 | 4 | 1 | 0.9 | IM | rhTSH in SWFI | Pre-dose, 30, 60, 90, 120, 150, 180, 240, 480, 1440, 1920 minutes |
| 2 | 5 | | | | rhTSH in 0.25% MC | |
| 3 | 5 | | | | rhTSH in 3.0% NaCMC | |

### Pharmacokinetic Parameters

Pharmacokinetic analysis was performed on individual animals. Pharmacokinetic analysis of serum concentration-time data was performed using WinNonlin^{®} computer software (Pharsight Corporation, Mountain View, CA). The terminal elimination half-life (t_{1/2}) represents the time required for the drug concentration at any point on the straight line (log-linear scale) to decrease by one-half is given by 0.693/n, where n is the elimination rate constant (the product of 2.303 and the terminal slope). The Cₘₐₓ is the maximum serum concentration in ng/mL. The Tₘₐₓ is the time at which maximum concentration was observed (time at Cₘₐₓ) in minutes. The Cₘₐₓ, Tₘₐₓ, terminal elimination half-life (t_{1/2}), area under the curve (AUC), and clearance (Cl) parameters are summarized in Table 4. FIG. 2 depicts the mean serum concentration (ng/mL) of TSH at particular timepoints after intramuscular administration (IM) of 0.25% MC or 3% NaCMC formulations of rhTSH in rats.

**Table 4: Cmax, Tmax, t1/2, AUC and Cl Values for SWFI, MC and NaCMC Formulations**

| | **Cₘₐₓ ng/mL** | **Tₘₐₓ min** | **t_{1/2} min** | **AUC (all) min*ng/mL** | **Cl mL/min/kg** |
|---|---|---|---|---|---|
| rhTSH in SWFI | 1162.48 ± 254.94 | 165.0 ± 17.32 | 252.90 ± 24.11 | 793094.70 ± 80246.60 | 1.28 ± 0.15 |
| rhTSH in 0.25% MC | 1107.82 ± 130.50 | 204.00 ± 32.86 | 250.74 ± 11.77 | 767464.14 ± 63019.42 | 1.28 ± 0.13 |
| rhTSH in 3% NaCMC | 701.64 ± 102.49 * | 384.00 ± 131.45 * | 354.32 ± 48.32 * | 661417.56 ± 80174.02 * | 1.48 ± 0.19 |

| | | | | | |
|---|---|---|---|---|---|
| * P value < 0.05 | | | | | |

### Summary of Pharmacokinetic Parameters

Following the bolus IM administration of each rhTSH preparation, the semilogarithmic plot of concentration versus time demonstrated an absorption, distribution and elimination profile consistent with route of administration. Under the conditions of this study, IM administration of rhTSH reconstituted in 3% NaCMC resulted in a statistically-significant shift of Cₘₐₓ, when compared to rhTSH in SWFI. There was no statistically-significant difference between modified-release rhTSH in 0.25 % MC as compared to rhTSH is SWFI.

### Example 4: Pharmacokinetics of rhTSH administered to rats:

### Effect of Viscosity and type of Sodium Carboxymethylcellulose

In this Example, the pharmacokinetics (PK) of six different formulations of rhTSH were compared. The study design consisted of 30 jugular vein cannulated rats divided into 6 groups. All rats were administered a single dose of 1 mg/kg recombinant human TSH (rhTSH) through intramuscular injection (IM). The 6 administration vehicles were sterile water for injection and different viscosities of sodium carboxymethylcellulose (NaCMC) at approximately 0.9 mg/mL. In particular:

Group 1 was administered rhTSH in sterile water for injection (SWFI);

Group 2 was administered rhTSH in 2% medium viscosity NaCMC from Hercules;

Group 3 was administered rhTSH in 1.5% medium viscosity NaCMC form Hercules;

Group 4 was administered rhTSH in 3% low viscosity NaCMC from Ruger;

Group 5 was administered rhTSH in 3% low viscosity NaCMC from Hercules; and

Group 6 was administered rhTSH in 2% low viscosity NaCMC from Hercules.

Serum samples were taken for PK analysis (n=3) at 0, 30, 60, 90, 120, 150, 180, 240, 480 and 1440 minutes. Serum samples were evaluated using a rhTSH ELISA.

### Materials and Methods

Experiments were performed as described in Example 2.

Table 5 provides a summary of the parameters for the study. No abnormalities in the animals were observed during the in-life portion of the study or at necropsy.

**Table 5: Study Design**

| Group # | # of Animals | Dose mg/kg | Conc mg/mL | Dose Route | Test Article | Viscosities | Time-points |
|---|---|---|---|---|---|---|---|
| 1 | 5 | 1 | 1 | IM | rhTSH in SWFI | NA | Pre-dose, 60,90, 120, 150, 180, 240, 480, 1440 minutes |
| 2 | 5 | | | | rhTSH in 2% medium viscosity NaCMC, Hercules | 149 cps | |
| 3 | 5 | | | | rhTSH in 1.5% medium viscosity NaCMC, Hercules | 79 cps | |
| 4 | 5 | | | | rhTSH in 3% low viscosity NaCMC, Ruger | 76 cps | |
| 5 | 5 | | | | rhTSH in 3% low viscosity NaCMC, Hercules | 46 cps | |
| 6 | 5 | | | | rhTSH in 2% low viscosity NaCMC, Hercules | 18 cps | |

### Pharmacokinetic Parameters

Pharmacokinetic analysis was performed on individual animals. Pharmacokinetic analysis of serum concentration-time data was performed using WinNonlin^{®} computer software (Pharsight Corporation, Mountain View, CA). The terminal elimination half-life (t_{1/2}) represents the time required for the drug concentration at any point on the straight line (log-linear scale) to decrease by one-half is given by 0.693/n, where n is the elimination rate constant (the product of 2.303 and the terminal slope). The Cₘₐₓ is the maximum serum concentration in ng/mL. The Tₘₐₓ is the time at which maximum concentration was observed (time at Cₘₐₓ) in minutes. The Cₘₐₓ, Tₘₐₓ, terminal elimination half-life (t_{1/2}), area under the curve (AUC), and clearance (Cl) parameters are summarized in Table 6. FIG. 3 depicts the mean serum concentration (ng/mL) of TSH at particular timepoints after intramuscular administration (IM) of various NaCMC formulations of rhTSH in rats.

**Table 6: Cmax, Tmax, t1/2, AUC and Cl Values for SWFI and NaCMC Formulations**

| | **Cₘₐₓ ng/mL** | **Tₘₐₓ min** | **t_{1/2} min** | **AUC (all) min*ng/mL** | **Cl mL/min/kg** |
|---|---|---|---|---|---|
| rhTSH in SWFI | 1869.40 ± 463.09 | 108.00 ± 40.25 | 208.79 ± 41.46 | 676612.77 ± 56319.96 | 1.66 ± 0.11 |
| rhTSH in 2% NaCMC 149 cps | 839.93 ± 109.93 * | 216.00 ± 32.86 * | 316.95 ± 75.24 * | 582323.73 ± 28661.78 * | 1.70 ± 0.23 |
| rhTSH in 1.5% NaCMC 79 cps | 1091.62 ± 145.52 * | 138.00 ± 26.83 | 320.97 ± 43.84 * | 543958.41 ± 87182.51 * | 1.8 ± 0.27 |
| rhTSH in 3% NaCMC Ruger | 1300.86 ± 233.36 * | 132.00 ± 16.43 | 252.75 ± 61.52 | 501049.86 ± 10301.70 * | 2.0 ± 0.17 * |
| rhTSH in 3% NaCMC Hercules | 1360.49 ± 394.57 | 120.00 ± 00 | 290.92 ± 33.71 * | 484258.29 ± 89921.25 * | 2.04 ± 0.38 |
| rhTSH in 2% NaCMC Hercules | 1370.54 ± 249.74 | 120.00 ± 0.00 | 261.62 ± 20.02 * | 579898.44 ± 50108.82 * | 1.78 ± 0.23 |

| | | | | | |
|---|---|---|---|---|---|
| * P value < 0.05 | | | | | |

### Summary of Pharmacokinetic Parameters

Following the bolus IM administration of each rhTSH preparation, the semilogarithmic plot of concentration versus time demonstrated an absorption, distribution and elimination profile consistent with route of administration. A student's T-test was performed comparing the WinNonLin^{®} parameters for each of the modified-release rhTSH samples to the rhTSH WinNonLin^{®} parameters. The Cₘₐₓ terminal half life (t ½), and the area under the curve (exposure) was statistically significantly different for all of the NaCMC vehicles tested indicating that changing the viscosity of administered rhTSH solution alters the pharmacokinetic parameters significantly.

Under the conditions of this study, the pharmacokinetic analysis demonstrated statistically-significant changes between rhTSH administered in NaCMC and rhTSH administered in SWFI at all viscosities tested.

### Example 5: Pharmacokinetics of rhTSH administered to rats:

### Effect of Viscosity/Concentration of Carboxymethylcellulose

In this Example, the pharmacokinetics (PK) of four different formulations of rhTSH were compared. The study design consisted of 40 jugular vein cannulated female Sprague-Dawley rats divided into 4 groups. Each group received a single intramuscular (IM) injection of the designated rhTSH formulation. In particular:

Group 1 received rhTSH at 0.1 mg/kg in sterile water for injection (SWFI);

Group 2 received rhTSH at 0.1 mg/kg in 2.5% carboxymethylcellulose (NaCMC) at 41 centipoise (cps);

Group 3 received rhTSH at 0.1 mg/kg in 3.0% NaCMC at 97 cps; and

Group 4 received rhTSH at 0.1 mg/kg in 3.5% NaCMC at 159 cps.

Following dosing all animals received serial bleed via the jugular vein cannula. All blood samples were processed for serum and the serum was transferred into corresponding labeled microcentrifuge tubes. All samples were stored at -80°C until time of analysis.

### Materials and Methods

Experiments were performed as described in Example 2.

Table 7 provides a summary of the parameters for the study. No abnormalities in the animals were observed during the in-life portion of the study or at necropsy.

**Table 7: Study Design**

| Group | # of Animals | Dose mg/kg | Conc. mg/ml | Test Article | Vehicle | Dosing Regimen | Dose Route |
|---|---|---|---|---|---|---|---|
| 1 | 10 | 0.1 | 0.25 | rhTSH | SWFI | Single Dose | IM |
| 2 | 10 | | | | 2.5% NaCMC | | |
| 3 | 10 | | | | 3.0% NaCMC | | |
| 4 | 10 | | | | 3.5% NaCMC | | |

### Pharmacokinetic Parameters

Pharmacokinetic analysis was performed for each animal using non-compartmental method with WinNonlin^{®} software version 5.0 (Pharsight Corp., Mountain View, CA). The slope (beta) of the terminal log-linear phase of each concentration versus time curve was determined by linear regression analysis. This slope was used to calculate the apparent elimination half-life. Area under the serum concentration curve (AUC) from time zero until the last detectable concentration was determined by the linear trapezoidal method and extrapolated to infinity. Apparent clearance was calculated as the administered dose of rhTSH divided by the total AUC, and the apparent volume of distribution was calculated as clearance divided by beta. Mean serum concentrations of rhTSH versus time curves for each test article are shown in FIG. 4. Pharmacokinetic parameters were averaged for each test article and are shown in Table 8.

**Table 8: Pharmacokinetic Parameters for rhTSH in Sprague Dawley Rats (0.1 mg/kg, IM) followed by a Dunnett's post hoc test**

| Pharmacokinetic Parameter (± SD) | rhTSH in SWFI (n=9) | rhTSH in 2.5% NaCMC (n=8) | rhTSH in 3.0% NaCMC (n=10) | rhTSH in 3.5% NaCMC (n=6) |
|---|---|---|---|---|
| t_{1/2} (min) | 273.08 ± 134.76 | 276.90 ± 60.95 | 347.45 ± 76.67 | 393.69 ± 96.81 |
| Cl (ml/min/kg) | 2.45 ± 0.85 | 2.27 ± 1.07 | 1.70 ± 0.18* | 2.34 ± 0.62 |
| Vz (ml/kg) | 871.70 ± 292.78 | 870.46 ± 297.81 | 855.86 ± 234.81 | 1346.52 ± 513.00* |
| Cₘₐₓ | 114.07 ± 39.30 | 70.22 ± 22.34* | 66.36 ± 11.19* | 59.69 ± 26.46* |
| Tₘₐₓ | 120.00 ± 70.36 | 210.00 ± 114.52* | 306.00 ± 153.49 | 155.00 ± 22.58 |
| AUC (ng*min/ml) | 45135.05 ± 13699.83 | 49994.38 ± 15527.05 | 59341.56 ± 6113.24 | 45278.48 ±11679.80 |

| | | | | |
|---|---|---|---|---|
| t_{1/2}, elimination half-life; Cl, clearance; Vz, apparent volume of distribution; Cₘₐₓ, maximum concentration; tₘₐₓ, time at which Cₘₐₓ observed; AUC, area under concentration curve extrapolated to infinity; *indicates p<0.05 compared to rhTSH in SWFI using a one-way analysis of variance | | | | |

### Summary of Pharmacokinetic Parameters

A one-way analysis of variance followed by a Dunnett's post hoc test was performed comparing the WinNonLin^{®} parameters for each of the modified-release TSH samples to the control rhTSH. PK analysis found there to be a statistically-significant difference in the mean Cₘₐₓ associated with all three concentrations of modified-release rhTSH, as compared to the control rhTSH. In addition, the data indicate that the mean t_{1/2} was different in the modified-release test articles. There was no statistically-significant difference between the mean AUCs for the 4 test articles, which suggests that the mean exposure in the modified-release rhTSH groups was not distinguishable from the mean exposure following administration of the control rhTSH.

These data confirm the observed results obtained from the other rat studies, namely, that there is a decrease in Cₘₐₓ and a shift in Tₘₐₓ to later time points with no change in the AUC (exposure). Specifically, IM administration of rhTSH administered in 2.5% (41 cps), 3% (97 cps) and 3.5% (159cps) NaCMC resulted in a significant shift of Cₘₐₓ, Tₘₐₓ, t_{1/2}, and AUC when compared to rhTSH administered in SWFI.

### Example 6: Pharmacokinetics of rhTSH administered to dogs:

### Effect of injection volume/rhTSH concentration

In this study, the pharmacokinetics (PK) of rhTSH administered to beagle dogs in 3% NaCMC and in SWFI were compared. The study design consisted of 15 dogs divided into three groups. All dogs were administered a single dose of 0.1 mg/kg rhTSH. In particular:

Group 1 was administered 0.15 ml/kg rhTSH in SWFI;

Group 2 was administered 0.15 ml/kg rhTSH in 3% NaCMC; and

Group 3 was administered 0.07 ml/kg rhTSH in 3% NaCMC.

Serum samples were taken for PK analysis (n=3) at 0, 15, 30, 60, 90, 120, 150, 180, 240, 300, 360, 480, 1440, and 2160 minutes. Serum samples were evaluated using the rhTSH ELISA.

### Methods and Materials

### rhTSH ELISA

A colorimetric sandwich enzyme linked immunosorbant assay (ELISA) using mouse anti-hCG antibody and Biotinylated mouse anti-rhTSH antibody was employed to quantitate the amount of rhTSH in dog serum. Plates were coated with mouse anti-hCG antibody and were incubated overnight at 2 to 8°C. A standard curve was prepared using rhTSH starting at 5.556 ng/ml and serially diluting 1:1.5 to 0.488 ng/ml. A series of dilutions were prepared for each test sample in dilution buffer. Standards, controls, and samples were added to the plates in duplicate and were incubated for one hour at 37°C with shaking. Plates were washed with ELISA plate wash solution. Biotinylated mouse anti-rhTSH was diluted appropriately in sample dilution buffer, added to each well, and incubated for one hour at 37°C with shaking. Plates were washed six times with ELISA plate wash solution. Streptavidin horseradish peroxidase was diluted appropriately in sample diluent buffer, was added to each well, and incubated for 15 minutes at room temperature in the dark. Plates were washed with ELISA plate wash solution. Ortho-phenylenediamine (OPD) was added and incubated for 30 minutes at 37°C with shaking. The reaction was stopped using a 4.5M sulfuric acid stop solution. The amount of rhTSH in each sample was measured at an absorbance reading of 490 nm.

### Data Analysis

Absorbance readings for samples were excluded if the absorbance measurement at 490 nm was greater than that achieved by the highest rhTSH standard (5.556 ng/ml), or less than the reading obtained for the lowest rhTSH standard (0.488 ng/ml). Such readings were not included in the data analysis.

The amount of rhTSH (measured in ng/ml) in each sample dilution was interpolated from the standard curve, multiplied by the appropriate dilution, and expressed as ng of rhTSH/ml of serum. The amount of rhTSH in each sample was calculated by multiplying the appropriate dilution factor by the interpolated result. The amount of rhTSH is expressed as ng of rhTSH/ ml of serum.

### WinNonLin^{®} Analysis

To obtain pharmacokinetic parameter estimates, serum rhTSH concentration-time data were analyzed using the nonlinear least squares curve-fitting program, WinNonlin^{®} (Pharsight Corporation, Mountain View, CA). The terminal elimination half-life (t_{1/2}) represents the time required for the drug concentration at any point on the straight line (log-linear scale) to decrease by one-half is given by 0.693/n, where n is the elimination rate constant (the product of 2.303 and the terminal slope). The Cₘₐₓ is the maximum serum concentration in ng/ml. The Tₘₐₓ is the time at which maximum concentration was observed (time at Cₘₐₓ) in minutes. The terminal elimination half-life (t_{1/2}), area under the curve (AUC), clearance, Cₘₐₓ, and Tₘₐₓ parameters are summarized.

Mean serum concentrations of TSH versus time curves for each test article are shown in FIG. 5. Pharmacokinetic parameters were averaged for each test article and are shown in Table 9.

### Conclusions

A student's T-test was performed comparing the WinNonLin^{®} parameters for each of the modified-release rhTSH samples to the rhTSH WinNonLin^{®} parameters. The analysis demonstrated a statistically-significant difference in the Cₘₐₓ and the Tₘₐₓ for both concentrations of rhTSH administered in 3% NaCMC, as compared to rhTSH administered in sterile water for injection. There was no significant difference in the AUC between groups, which suggests that the exposure was not different for the three groups. It was also noted that there was a higher Cₘₐₓ in the group 3 animals, which was consistent with a higher concentration of rhTSH in this dose group. The results from this study demonstrated a decrease in Cₘₐₓ and a shift in Tₘₐₓ for the rhTSH administered in 3% NaCMC as compared to rhTSH administered in sterile water for injection.

**Table 9: Pharmacokinetic Parameters for rhTSH in Beagles (0.1 mg/kg, IM)**

| | **Cₘₐₓ ng/mL** | **Tₘₐₓ min** | **AUC (all) min*ng/mL** |
|---|---|---|---|
| **Thyrogen^{®}** | 935.32 ± 339.61 | 66.00 ± 25.10 | 321186.80 ± 79832.95 |
| **MRF rhTSH (.7 mg/mL)** | 384.10 ± 205.05 * | 234.00 ± 68.41 * | 248291.40 ± 55235.03 |
| **MRF rhTSH (1.43 mg/mL)** | 513.60 ± 113.27* | 180.00 ± 60.00* | 297390.20 ± 49397.20 |

| | | | |
|---|---|---|---|
| * P value < 0.05 | | | |

### Example 7: Pharmacokinetics of rhTSH administered to dogs:

### Effect of dose and viscosity of the NaCMC vehicle

In this study, the pharmacokinetics (PK) of rhTSH administered in 3% NaCMC were compared to the PK of rhTSH administered in SWFI in beagle dogs following a single intramuscular (IM) injection. Two different viscosities of the viscous vehicle were tested, 93 cps and 54 cps. The study design consisted of 35 dogs divided into seven groups, n=5 per group. Dogs were administered test article at either, 0, 0.05, 0.1, or 0.2 mg/kg. In particular:

Group 1 was administered 0.05 mg/kg rhTSH in SWFI;

Group 2 was administered 0.05 mg/kg rhTSH in 3% NaCMC at 93 cps;

Group 3 was administered 0.1 mg/kg rhTSH in SWFI;

Group 4 was administered 0.1 mg/kg rhTSH in 3% NaCMC at 93 cps;

Group 5 was administered 0.1 mg/kg rhTSH in 3% NaCMC at 54 cps;

Group 6 was administered 0.2 mg/kg rhTSH in SWFI; and

Group 7 was administered 0.2 mg/kg rhTSH in 3% NaCMC at 93 cps.

Serum samples were taken for PK analysis (n=3) at 0, 15, 30, 60, 90, 120, 150, 180, 240, 300, 360, 480, and 1440 minutes. Serum samples were evaluated using the rhTSH ELISA.

### Materials and Methods

Experiments were performed as described in Example 6.

Mean serum concentrations of TSH versus time curves for the test articles are shown in FIGS. 6, 7 and 8. Pharmacokinetic parameters were averaged for each test article and are shown in Table 10.

### Conclusions

A student's T-test was performed comparing the WinNonLin^{®} parameters for each of the rhTSH administered in NaCMC samples to the WinNonLin^{®} parameters of rhTSH administered in SWFI. The analysis demonstrated that there was a statistically-significant difference in Cₘₐₓ, Tₘₐₓ and t_{1/2} for the rhTSH administered in 3% NaCMC at 0.05 mg/kg when compared to the rhTSH administered in sterile water for injection at the same dose. In addition, the analysis demonstrated that there was a statistically-significant difference in Cₘₐₓ, Tₘₐₓ and t_{1/2} for the rhTSH administered in 3% NaCMC at 0.1 mg/kg (at 54 and 93 cps) when compared to the rhTSH administered in sterile water for injection at the same dose. There was no difference in the pharmacokinetic parameters of rhTSH administered in 3% NaCMC at 54 cps when compared to rhTSH administered in 3% NaCMC at 93 cps. Finally, the analysis demonstrated that there was a statistically-significant difference in Cₘₐₓ, Tₘₐₓ and t_{1/2} of the rhTSH administered in 3% NaCMC at 0.2 mg/kg when compared to the rhTSH administered in sterile water for injection at the same dose. However, at this higher dose there was a statistically-significant decrease in the AUC with a statistically-significant increase in clearance at this dose.

**Table 10: Pharmacokinetic Parameters for rhTSH in Beagles (0.1 mg/kg, IM)**

| | **Cₘₐₓ ng/mL** | **Tₘₐₓ min** | **t_{1/2} min** | **AUC (all) min*ng/mL** | **Cl mL/min/ kg** |
|---|---|---|---|---|---|
| rhTSH in SWFI (0.05 mg/kg) | 493.42 ± 75.52 | 54.00 ± 13.42 | 295.10 ± 21.16 | 120365.86 ± 14206.33 | 0.41 ± 0.05 |
| rhTSH in 3% NaCMC (0.05 mg/kg) | 201.48 ± 13.15 * | 228.00 ± 88.99 * | 353.87 ± 33.83 * | 128704.08 ± 19319.39 | 0.37 ± 0.05 |
| rhTSH in SWFI (0.1 mg/kg) | 840.80 ± 308.09 | 45.00 ± 21.21 | 310.14 ± 28.30 | 224424.46 ± 86019.43 | 0.54 ± 0.37 |
| rhTSH in 3% NaCMC (0.1 mg/kg, 93 cps) | 340.56 ± 40.04 * | 252.00 ± 26.83 * | 378.92 ± 35.30 * | 221390.86 ± 40849.17 | 0.43 ± 0.08 |
| rhTSH in 3% NaCMC (0.1 mg/kg, 54 cps) | 377.06 ± 82.13 * | 240.00 ± 0.00 * | 409.96 ± 74.51 * | 209986.36 ± 61713.95 | 0.46 ± 0.13 |
| rhTSH in SWFI (0.2 mg/kg) | 1725.00 ± 486.87 | 54.00 ± 13.42 | 339.68 ± 69.77 | 466176.48 ± 73706.03 | 0.42 ± 0.07 |
| rhTSH in 3% NaCMC (0.2 mg/kg, 93 cps) | 506.30 ± 197.68 * | 157.50 ± 66.52 * | 400.03 ± 166.72 | 231956.63 ± 73681.09 * | 0.87 ± 0.42 * |

| | | | | | |
|---|---|---|---|---|---|
| *P value < 0.05 | | | | | |

### Example 8: Pharmacokinetics of rhTSH administered to Humans

This study assessed and compared the pharmacokinetics of a single intramuscular (IM) administration of 0.1 mg of Thyrogen^{®} versus a single IM administration of 0.1 mg of a modified-release rhTSH (MRrhTSH) formulation in 3% sodium carboxymethylcellulose (NaCMC) in healthy human subjects. The study further assessed and compared the safety profile, the pharmacodynamic (PD) effects, and the thyroid uptake of radioiodine (¹²³I) following a single IM administration of 0.1 mg of Thyrogen^{®} versus a single dose of 0.1 mg of a MRrhTSH formulation in 3% NaCMC in healthy human subjects.

### Methodology and Subjects

This study had a single-dose, randomized, single-blind, parallel-group, comparative bioavailability design. A total of 46 healthy adult subjects were to be randomly assigned to receive a single dose of Thyrogen^{®}, 0.1 mg IM, or a single dose of MRrhTSH in 3% NaCMC, 0.1 mg IM. A total of 46 subjects were planned; 46 subjects were enrolled; and 45 subjects received study drug and were analyzed for safety, pharmacokinetics, and pharmacodynamics.

A subgroup of subjects (n=10) received a tracer dose of radioiodine (approximately 400 µCi ¹²³I) so that radioiodine uptake (RAIU) by the thyroid gland could be measured. Results for 1 subject in the RAIU group who withdrew prior to receiving study drug were listed, but were not included in summary statistics.

Screening evaluations were performed as follows: For all subjects, within 14 days prior to study drug administration; and for the RAIU subgroup, within 14 days prior to baseline RAIU measurements, which were performed within the week prior to study drug administration.

Subjects checked into the clinic on the evening prior to dosing and were confined to the clinic for 96 hours after dosing. Subjects fasted overnight for at least 10 hours prior to dosing. Serial serum samples were collected for determination of thyroid stimulating hormone (TSH) concentration levels during the 336 hours following administration of study medication.

### Diagnosis and Main Criteria for Inclusion

Healthy adult male or female subjects with a body mass index (BMI) <30 kg/m² and normal physical examination, vital signs, laboratory assessments, electrocardiogram (ECG), and Holter results at the time of screening. Subjects with non-thyroidal conditions known to affect ¹²³I uptake (e.g., congestive heart failure class III or IV, renal failure) or those currently taking drugs that may affect thyroid or renal function (e.g., corticosteroids, diuretics, lithium, amiodarone, or other prescribed iodine-containing medication) were to be excluded.

### Formulations

Subject were administered Thyrogen^{®} or a MRrhTSH formulation in 3% NaCMC, which contained the same active drug substance but was reconstituted in a different diluent. Both formulations were formulated in a 20 mM sodium phosphate buffer, pH 7.0, containing 0.2% sodium chloride prior to lyophilization. The quantitative composition of the lyophilized drug per vial was 1.1 mg thyrotropin alfa, 36 mg mannitol, 1.4 mg sodium phosphate (monobasic, monohydrate), 3.7 mg sodium phosphate (dibasic, heptahydrate), and 2.4 mg sodium chloride.

Thyrogen^{®} was reconstituted with 5.5 ml sterile water for injection (SWFI), European Pharmacopeia (EP)/ United States Pharmacopeia (USP). MRrhTSH was reconstituted with 5.5 ml sterile, non-pyrogenic solution of 3% NaCMC in SWFI, E/PUSP. The diluent for the MRrhTSH, 3% NaCMC, was supplied in vials as a sterile liquid, and was stored at 2 to 8°C (36 to 46°F). All study medication and diluent was brought to room temperature (approximately 30 minutes) before preparation. The contents of the study medication vial were vented and reconstituted, using aseptic technique. The study medication was mixed by inversion and gentle swirling until the lyophilized product was dissolved. After reconstitution, the concentration of thyrotropin alfa in both study medications was 0.2 mg/mL. Each vial of study medication was intended for single use.

### Safety

Safety was evaluated through the monitoring of adverse events (AEs), clinical laboratory tests, vital sign measurements, physical examinations, ECGs, Holter monitoring, thyroid ultrasounds, and TSH levels. Treatment was well tolerated by the subjects in this study. Few AEs were reported, and the incidences in the 2 treatment groups were the same (3 subjects each, 13%). The events considered to be related to study drug included abdominal pain, diarrhea, dizziness, headache, and hypertension (all reported by 1 subject each). All AEs were mild or moderate and none led to study discontinuation.

Consistent with the effect of multiple blood-sampling procedures in this study, mean values for many of the hematology parameters (hemoglobin, hematocrit, RBC, WBC, neutrophils, and lymphocytes) trended lower at the end of the study. However, no apparent treatment-related trends were observed in the analysis of laboratory results.

On Day 4, there were 3 subjects who had ECG abnormalities which were not clinically significant. Otherwise, results of the 12-lead ECG, Holter monitoring, and thyroid ultrasound assessments did not reveal any new safety issues, and no treatment-related trends were discerned.

### Statistical Methods

All analyses were performed using Statistical Analysis System (SAS), Version 8.2 or higher. Main effects were tested at the 0.05 level.

### Pharmacokinetic Analysis

The following pharmacokinetic (PK) parameters were calculated for TSH: maximum observed drug concentration (Cₘₐₓ), time to reach Cₘₐₓ (Tₘₐₓ), last measurable concentration (Cₗₐₛₜ), smallest disposition rate constant (λ_{z}), elimination half-life (t_{1/2}), area under the concentration-time curve from time 0 to time of Cₗₐₛₜ (AUC₀₋ₜ), and AUC from time 0 to infinity (AUC0-∞). PK parameters were calculated for TSH using standard, non-compartmental methods with and without baseline correction for endogenous TSH. Calculations were performed using WinNonlin^{®} (Version 4.1) computer software (Pharsight Corporation, Mountain View, CA).

The baseline TSH value for a given subject was the average of the time 12 hour and time 0 hour (pre-dose) TSH values. Individual concentration time points that were <0 after baseline subtraction were set to 0 for estimation of PK variables and calculation of descriptive statistics. PK parameters, individual subject TSH concentrations, and actual sampling times were listed and summarized using descriptive statistics by treatment group with and without baseline correction.

Differences in PK parameters were assessed by examining mean and median values of Cₘₐₓ, Tₘₐₓ, t_{1/2}, AUC₀₋ₜ, and AUC0-∞ between treatment groups for all subjects and the RAIU subgroup. Following log-transformation (natural log), AUCO-oo, AUC₀₋ₜ, and Cₘₐₓ results were compared between treatment groups using p-values and 90% confidence intervals (CIs) around the geometric mean ratio. PROC MIXED in SAS® (Version 8.2) was used with treatment as a fixed effect in the model as follows:

### PK VARIABLE ESTIMATE = TREATMENT

### Pharmacodynamic Analysis and Determination of Levels of TSH, T₃, free T₃, T₄, and free T₄

Thyroid function tests (TFTs) included measurement of free thyroxine (T₄), total T₄, free triiodothyronine (T₃), and total T₃ levels. Cₘₐₓ and Tₘₐₓ were determined for these parameters. RAIU by the thyroid gland following receipt of a tracer dose of 400 µCi ¹²³I was determined in a subset of subjects.

TSH concentrations and levels of T₃, free T₃, T₄, and free T₄ were determined from serum samples using a validated Direct Chemiluminescent Assay method performed on the Bayer ADVIA Centaur Analyzer. The assay is an FDA-approved assay for *in vitro* diagnostic testing. The instrument has volumes of assay precision runs, calibrations, verifications and linearity runs that are required before an instrument can perform an assay.

Results of TFTs (free T₃, total T₃, free T₄, and total T₄) were listed and summarized by treatment group using descriptive statistics. Results were assessed for both efficacy (a slight rise) and safety (the absence of a large increase) by examining Cₘₐₓ and Tₘₐₓ. These results were analyzed by examining differences in mean and median Cₘₐₓ and Tₘₐₓ between treatment groups using a t-test and the Wilcoxon rank sum test.

### Radioiodine Uptake

For the RAIU subgroup, determination and comparison of the increase in RAIU by the thyroid gland from a baseline evaluation was made following administration of study medication. The absolute values and relative effect of treatment on RAIU were summarized. The mean, standard deviation, median, range of baseline uptake, post-treatment uptake, change in uptake from baseline to post-treatment, and percent change in uptake from baseline to post-treatment were calculated at each time point. Similarly, the ratio of post-treatment uptake:baseline uptake at each time point was summarized for both treatment groups.

### Results

### Pharmacokinetic Analysis

PK was assessed for 23 subjects in the Thyrogen^{®} group and 22 subjects in the MRrhTSH group. As was observed in this study, administration of exogenous TSH resulted in a rapid increase in plasma TSH and a consequent decrease in TSH levels with a rebound or 'overshoot' of plasma TSH gradually above baseline late in the plasma concentration profile. These increased TSH concentrations due to endogenous TSH can confound the PK estimates for exogenous administered drug. Therefore, the data in this study were presented with and without baseline correction. Following dosing, corrected values that fell below zero (and all subsequent assessments) were set to missing for the estimation of PK variables. In this study, the majority of subjects reached this point at 96 hours following dosing.

The AUC_{0-∞} estimated from uncorrected plasma concentration data could not be estimated due to rising TSH concentrations at later time points. Likewise, the AUC₀₋ₜ measured exposure to endogenous and exogenous TSH, and in particular, reflected TSH exposure due to endogenous rebound late in the profile. To better quantify TSH exposure from exogenously administered TSH, partial AUCs were calculated from time 0 to 24, 48, 72, and 96 hours after dosing.

At baseline, plasma concentrations were comparable between treatments, with mean concentrations of 1.69 and 1.63 µIU/mL TSH for the Thyrogen^{®} and MRrhTSH treatment groups, respectively. Mean TSH concentrations increased following injection of study drug and gradually declined over the next 4 days, with mean concentrations falling below baseline levels at approximately 96 hours following dosing. Consistent with the change in formulation, maximum TSH concentrations for the MRrhTSH treatment group were significantly lower, maximum TSH concentrations were achieved approximately 3 hours later, and TSH levels declined at a slower rate than those found after dosing with the Thyrogen^{®} control formulation.

### Uncorrected Data

At baseline, plasma concentrations were comparable between treatments, with mean concentrations of 1.69 and 1.63 µIU/mL TSH for the Thyrogen^{®} and MRrhTSH treatment groups, respectively. Mean TSH concentrations increased following injection of study treatment and gradually declined over the next 4 days, with mean concentrations falling below baseline levels at approximately 96 hours following dosing. Maximum TSH concentrations were lower for the MRrhTSH treatment group and were achieved approximately 3 hours later than those of the Thyrogen^{®} group. Following Tₘₐₓ, the decline in plasma TSH appeared more gradual for subjects in the modified-release formulation compared to the Thyrogen^{®} control formulation. This more gradual decline was due to the continued and prolonged absorption of TSH from the modified-release formulation injection site.

TSH PK parameters were estimated using uncorrected TSH concentrations and baseline-corrected concentrations. As the t_{1/2} and AUC0-∞ cannot be estimated for the uncorrected plasma data, the uncorrected PK values included AUC₀₋ₜ, Cₘₐₓ, and Tₘₐₓ. In order to assess the impact of the increasing TSH concentrations after the 96-hour time point, additional partial AUC variables were estimated from uncorrected data, including AUC₀₋₂₄, AUC₀₋₄₈, AUC₀₋₇₂, and AUC₀₋₉₆.

Results of the uncorrected PK parameters show that the bioavailability of the MRrhTSH treatment was lower than that of Thyrogen^{®} at all time intervals, but the values showed a trend toward convergence over time (Table 11).

**Table 11: Arithmetic Mean Uncorrected TSH Pharmacokinetic Parameters**

| **PK Parameter** | **Thyrogen^{®}** | | **MRrhTSH** | |
|---|---|---|---|---|
| | **N** | **Mean (SD)** | **N** | **Mean (SD)** |
| Cₘₐₓ (µIU/mL) | 23 | 26.26 (7.93) | 22 | 17.31 (14.04) |
| Tₘₐₓ (hr) [1] | 23 | 6.00 (3.00, 9.00) | 22 | 9.00 (5.00, 337.4) |
| AUC₀₋ₜ (µIU*hr/mL) | 23 | 948.30 (250.94) | 22 | 991.04 (486.04) |
| AUC₀₋₂₄ (µIU*hr/mL) | 23 | 381.48 (89.21) | 22 | 271.17 (203.26) |
| AUC₀₋₄₈ (µIU*hr/mL) | 23 | 544.22 (99.29) | 22 | 464.79 (263.52) |
| AUC₀₋₇₂ (µIU*hr/mL) | 23 | 598.82 (97.90) | 22 | 560.98 (268.39) |
| AUC₀₋₉₆ (µIU*hr/mL) | 23 | 623.14 (98.51) | 22 | 608.10 (269.97) |
| [1] Median (min, max) | | | | |

Mean uncorrected plasma TSH concentrations through the first 96 hours following dosing are plotted on a linear scale by treatment in FIG. 9.

### Baseline-Corrected Data

Plasma concentrations were measured for 336 hours following dosing. As was observed for the uncorrected data, mean TSH concentrations reached a peak at approximately 6 to 9 hours following injection of study treatment and gradually declined to below baseline levels at approximately 96 hours following dosing. After the 96-hour time point, mean baseline-corrected concentrations increased above baseline levels.

The arithmetic mean TSH PK parameters estimated using baseline-corrected TSH concentrations are presented in Table 12.

**Table 12: Arithmetic Mean Baseline-Corrected TSH Pharmacokinetic Parameters**

| **PK Parameter** | **Thyrogen^{®}** | | **MRrhTSH** | | |
|---|---|---|---|---|---|
| | **N** | **Mean (SD)** | **N** | **Mean (SD)** | **p-value** |
| AUC0-∞ (µIU*hr/mL) | 22 | 505.81 (115.09) | 17 | 502.44 (297.40) | 0.3078 |
| AUC₀₋ₜ (µIU*hr/mL) | 23 | 474.50 (134.03) | 22 | 464.42 (282.96) | 0.2867 |
| Cₘₐₓ (µIU/mL) | 23 | 24.58 (7.84) | 22 | 15.68 (14.24) | 0.0004 |
| Tₘₐₓ (hr)[1] | 23 | 6.00 (3.00, 9.00) | 22 | 9.00 (5.00, 24.00) | 0.0003 |
| λ_{z} (hr⁻¹) | 22 | 0.0702 (0.0190) | 17 | 0.0527 (0.0191) | 0.0042 |
| t_{1/2} (hr) | 22 | 9.88 (2.69) | 17 | 13.17 (4.93) | 0.0042 |
| p-value by Wilcoxon rank-sum test [1] Median (min, max) | | | | | |

Mean Cₘₐₓ was approximately 57% higher for the Thyrogen^{®} treatment, with a mean value of 24.58 µIU/mL TSH compared to a Cₘₐₓ of 15.68 µIU/mL TSH for the MRrhTSH treatment. Peak concentrations were achieved at median times of 6 hours and 9 hours for the Thyrogen^{®} and MRrhTSH groups, respectively. Despite the lower peak concentrations, the longer t_{1/2} for the MRrhTSH group (13.17 hours versus 9.88 hours) resulted in AUC values that were comparable to those of the Thyrogen^{®} group. Mean AUC₀₋ₜ values for the MRrhTSH and Thyrogen^{®} groups were comparable at 464.42 µIU*hr/mL and 474.50 µIU*hr/mL, respectively.

### Analysis of Relative Bioavailability

The analysis of relative bioavailability was conducted on the log-transformed PK parameters of AUC and Cₘₐₓ. The baseline-corrected results demonstrate that total exposure (i.e., AUC) was approximately 10 to 13% lower for the modified-release formulation, with ratios (90% CI) of 0.8987 (0.7334, 1.1012; p=0.3810) and 0.8739 (0.6858, 1.1137; p=0.3553) for AUC_{0-∞} and AUC₀₋ₜ, respectively. The mean Cₘₐₓ following administration of the modified-release formulation was approximately 50% lower than that found following administration of Thyrogen^{®}, with a ratio of 0.5237 and 90% CI of 0.3999, 0.6859, which was statistically significant (p=0.0002).

**Table 13: Analysis of Relative Bioavailability: Baseline-Corrected and Uncorrected Pharmacokinetic Data**

| Parameter | Test MRrhTSH | Reference Thyrogen^{®} | Ratio[1] | 90% CI | p-value |
|---|---|---|---|---|---|
| Corrected Values | | | | | |
| AUC_{0-∞} (µIU*hr/mL) | 443.72 | 493.73 | 0.8987 | 0.7334, 1.1012 | 0.3810 |
| AUC₀₋ₜ (µIU*hr/mL) | 396.69 | 453.92 | 0.8739 | 0.6858, 1.1137 | 0.3553 |
| Cₘₐₓ (µIU/mL) | 12.24 | 23.36 | 0.5237 | 0.3999, 0.6859 | 0.0002 |
| | | | | | |

| Uncorrected Values | | | | | |
|---|---|---|---|---|---|
| AUC₀₋₂₄ (µIU*hr/mL) | 232.97 | 371.48 | 0.6271 | 0.5122, 0.7679 | 0.0004 |
| AUC₀₋₄₈ (µIU*hr/mL) | 418.55 | 535.59 | 0.7815 | 0.6581, 0.9280 | 0.0202 |
| AUC₀₋₇₂ (µIU*hr/mL) | 514.79 | 591.21 | 0.8707 | 0.7428, 1.0207 | 0.1503 |
| AUC₀₋₉₆ (µIU*hr/mL) | 562.50 | 615.71 | 0.9136 | 0.7845, 1.0639 | 0.3241 |
| AUC₀₋ₜ (µIU*hr/mL) | 909.41 | 919.77 | 0.9887 | 0.8382, 1.1663 | 0.9087 |
| Cₘₐₓ (µIU/mL) | 14.28 | 25.08 | 0.5692 | 0.4491, 0.7214 | 0.0002 |
| p-value from the PROC Mixed model [1] Ratio of the geometric least square (LS) mean of test to reference treatments. | | | | | |

PK results for the uncorrected data were comparable to those of the corrected data, with a Cₘₐₓ ratio of 0.5692 and p=0.0002. Results for the partial AUC converged over the post-dosing interval, with a ratio of 0.6271 for AUC₀₋₂₄ (p= 0.0004) and a ratio of 0.9136 for AUC₀₋₉₆ (p=0.3241). While results for AUC₀₋ₜ met the criteria for bioequivalence, with a ratio (90% CI) of 0.9887 (0.8382, 1.1663; p=0.9087), the estimate of this AUC parameter is confounded by endogenous release of TSH secondary to the hypothalamus-pituitary-thyroid axis response late in the PK profile and does not accurately represent drug released from the dosage form.

Consistent with the reduction in Cₘₐₓ found after dosing with the modified-release formulation, the difference in Tₘₐₓ between the 2 formulations was also statistically significant (p=0.0003 and 0.0009), by the Wilcoxon rank-sum test and t-test, respectively. Mean Tₘₐₓ (SD) values for the MRrhTSH and Thyrogen^{®} treatment groups were 10.73 (5.70) hours and 5.92 (2.06) hours, respectively.

Consistent with the difference in formulation, the plasma profile of MRrhTSH was characterized by lower peak TSH concentrations and slower rates of absorption and elimination compared to those observed with the Thyrogen^{®} control formulation. While the mean baseline-corrected mean Cₘₐₓ following administration of MRrhTSH was approximately 40% lower than Thyrogen^{®} (15.68 µIU/mL TSH versus 24.58 µIU/mL TSH), the bioavailabilities of the two formulations were roughly comparable, with ratios (90% CI) of 0.8987 (0.7334, 1.1012) and 0.8739 (0.6858, 1.1137) for AUC_{0-∞} and AUC₀₋ₜ.

At baseline, plasma TSH concentrations were comparable between treatments, with mean concentrations of 1.69 and 1.63 µIU/mL for the Thyrogen^{®} and MRrhTSH treatment groups, respectively. Following dosing, maximum TSH concentrations were lower for the MRrhTSH treatment group and were achieved approximately 3 hours later than those of the Thyrogen^{®} control group. Mean TSH concentrations in the MRrhTSH group increased following injection and gradually declined over the next 4 days, with mean concentrations falling below baseline levels at approximately 96 hours following dosing. The decline in plasma TSH appeared more gradual following administration of the modified-release formulation due to the continued and prolonged absorption of TSH from the modified-release formulation injection site.

Mean Cₘₐₓ (baseline corrected) was approximately 40% lower for the MRrhTSH treatment, with a mean value of 15.68 µIU/mL TSH compared to a Cₘₐₓ of 24.58 µIU/mL TSH for the Thyrogen^{®} treatment. Peak concentrations were achieved at median times of 6 hours and 9 hours for the Thyrogen^{®} and MRrhTSH groups, respectively. Despite the lower peak concentrations, the longer t_{1/2} for the MRrhTSH group (13.17 hours versus 9.88 hours) resulted in AUC values that were comparable to those of the Thyrogen^{®} group. Mean AUC₀₋ₜ values for the MRrhTSH and Thyrogen^{®} groups were 464.42 µIU*hr/mL and 474.50 µIU*hr/mL, respectively.

The analysis of relative bioavailability using baseline-corrected data demonstrated that injection with 0.1 mg MRrhTSH resulted in a decrease in the rate of absorption, with a Cₘₐₓ ratio of 0.5237 and a 90% CI of 0.3999 to 0.6859 (p=0.0002). The difference in Tₘₐₓ between the two treatments was also found to be statistically significant (p=0.0003). Overall bioavailability was lower for the modified-release formulation, with ratios (90% CI) of 0.8987 (0.7334, 1.1012; p=0.3810) and 0.8739 (0.6858, 1.1137; p=0.3553) for baseline-corrected AUC_{0-∞} and AUC₀₋ₜ.

Results for the uncorrected data demonstrated a Cₘₐₓ ratio of 0.5692 and a 90% CI that also excluded 1.0. Results for the partial AUC demonstrated a convergence of values over the post-dosing interval, with a ratio of 0.6271 for AUC₀₋₂₄ and a ratio of 0.9136 for AUC₀₋₉₆. The differences between treatments for the AUC₀₋₂₄ and AUC₀₋₄₈ were statistically significant (p=0:0004 and 0.0202, respectively), while the results for the remaining intervals of 0-72 and 0-96 hours were not significantly different (p=0.1503 and 0.3241, respectively).

### Pharmacodynamics

The effects of single 0.1 mg doses of Thyrogen^{®} and MRrhTSH on the concentrations of free T₃, total T₃, free T₄, and total T₄ were comparable between treatments when measured as early as 24 hours after the injection, with mean peak concentrations observed from 24 to 48 hours, followed by a steady decline to baseline levels by approximately 168 hours following dosing. In general, peak concentrations represented 1.5- to 2-fold increases above baseline levels. It was decided to retrieve frozen sera to assess total T₃ levels at earlier times after the injection, and at these earlier times, as described below, serum levels of total T₃ increased more gradually after MRrhTSH than after Thyrogen^{®}.

The results for percent RAIU at baseline were comparable between treatments, with respective mean uptake values of 10.82 and 10.86, 23.70 and 25.72, and 24.32 and 27.30 for the Thyrogen^{®} and MRrhTSH groups at 6, 24, and 48 hours following ¹²³I administration. Following treatment with Thyrogen^{®} or MRrhTSH, RAIU increased at a comparable rate in the 2 treatments, with increases of 225% and 277%, respectively at 6 hours, 125% and 123% at 24 hours, and 126% and 122% at 48 hours after administration of the ¹²³I dose.

### Analysis of T₃ (Triiodothyronine) Levels in Thyrogen^{®}-treated and MRrhTSH-treated Human Subjects

Both Thyrogen^{®} and MRrhTSH were well-tolerated by healthy subjects, although Holter monitoring revealed slight increases in the mean heart rates of both treatment groups on Day 3, about 48 hours after study drug was administered (from 72.7 to 78.2 bpm for all Thyrogen^{®} patients and from 72.9 to 79.0 bpm for all MRrhTSH patients at Screening and Day 3, respectively). Heart rate increases could be a safety concern, especially among older and sicker patients. It should be noted that the healthy subjects in this study did not complain about this change in heart rate, and they experienced no cardiac-related symptoms. Additional analyses of the mean heart rate data showed that the rise in mean heart rate was apparent on Day 2 (24 hours after study drug) in the Thyrogen^{®} subjects (mean of 76.0 bpm), but not in the MRrhTSH subjects (mean of 72.8 bpm). This acute effect in the first 24 hours may have been due to a more rapid release of triiodothyronine (T₃) from the thyroid following Thyrogen^{®} administration than following MRrhTSH administration. Rapid elevation of serum T₃ during the absorption phase after oral triiodothyronine or after desiccated thyroid (which contains both T₄ and T₃) are administered has reportedly caused palpitations, irritability, nervousness, dizziness, and tremor during thyroid hormone replacement therapy (Smith, R.N., et al., Br. Med. J. 4:145-48 (1970); Wiersinga, W.M., Horm. Res. 56(S1):74-81 (2001), Siegmund, W., et al., Clin. Endocrinol. 60:750-57 (2004)).

In order to better understand the possible relationship between T₃ and heart rate, total serum T₃ levels in the human subjects were measured using stored serum samples at multiple time points between 12 hours pre dose and 24 hours post dose. For all 45 subjects who received study medication, stored serum from the -12-hour, 0 minute, 2, 3, 4, 5, 6, 8, 12, and 24 hour time points were retrieved and assessed. Total T₃ was measured in each serum sample using the same laboratory, instrumentation, total T₃ assay, and methodology used for the total T₃ testing that had been performed earlier. Retesting of the -12 hour, 0 minute, and 24 hour samples allowed for a quality check, and T₃ levels that had been previously measured were indeed found again during retesting of the samples.

In brief, the baseline mean serum T₃ levels in the Thyrogen^{®} subjects (n = 23) and MRrhTSH subjects (n = 22) were the same. Mean serum T₃ levels trended higher in the Thyrogen^{®} group by 2 hours and remained higher over 24 hours than in the MRrhTSH group. Mean T₃ levels differed significantly between the 2 treatment groups at 3, 4, and 6 hours (p< 0.05) (FIG. 11). Thus, patients who receive MRrhTSH rather than Thyrogen^{®} would have a slower increase in serum T₃ level during the first 24 hours after administration, which may result in a lower likelihood of acutely-increased heart rate and may be an important consideration for some patients.

### Discussion

In brief, this study demonstrated that key PK parameters of MRrhTSH were different from Thyrogen^{®} when administered to human subjects. By adjusting the formulation to a higher viscosity, MRrhTSH had a delayed time to maximum concentration (Tmax). After IM injection of 0.1 mg Thyrogen^{®}, the Tmax of serum TSH occurred at approximately 6 hours, whereas after IM injection of 0.1 mg MRrhTSH the Tmax occurred at approximately 9 hours. The maximum plasma concentration (Cmax) of serum TSH (approximately 14 mU/L) was about 33% less after administration of MRrhTSH as compared to administration of Thyrogen^{®} (Cmax approximately 21 mU/L), but the area under the curve (AUC) of serum TSH (from the time of injection to 96 hours) was only slightly less for MRrhTSH. Both MRrhTSH and Thyrogen^{®} were pharmacodynamically active, as demonstrated by stimulation of 24-hour radioiodine uptake by the thyroid and by transient increases in serum thyroid function tests. Both drugs were generally well tolerated and were safe at a dose of 0.1 mg IM.

Thus, this study demonstrated the following:
i) the mean peak concentration for the MRrhTSH formulation was approximately 40% lower than that of the Thyrogen^{®} control formulation;
ii) the relative bioavailability of the modified-release formulation, as measured by total exposure, was approximately 10 to 13% lower than that of the Thyrogen^{®} control formulation;
iii) the difference in Tₘₐₓ between the modified-release formulation and the Thyrogen^{®} control formulation was statistically significant;
iv) following treatment with Thyrogen^{®} or MRrhTSH, RAIU increased at a comparable rate between treatments; and
v) administration of a single injection of 0.1 mg MRrhTSH did not raise any new safety concerns.

## Claims

1. A pharmaceutical composition comprising TSH and a pharmaceutically acceptable polymer that allows modified release of the TSH into a bloodstream of a patient, wherein the polymer is a cellulose derivative and when administered to the patient, the pharmaceutical composition provides an effective Tₘₐₓ of TSH in a serum of the patient that is at least about 20% longer than an effective Tₘₐₓ of TSH in the serum of the patient when a corresponding aqueous solution of TSH is administered.

2. A pharmaceutical composition comprising TSH and a pharmaceutically acceptable polymer that allows modified release of the TSH into a bloodstream of a patient, wherein the polymer is a cellulose derivative and when administered to the patient, the pharmaceutical composition provides an effective Cₘₐₓ of TSH in a serum of the patient that is at least about 20% lower than an effective Cₘₐₓ of TSH in the serum of the patient when a corresponding aqueous solution of TSH is administered.

3. A pharmaceutical composition comprising an effective amount of TSH and an effective amount of a pharmaceutically acceptable polymer wherein the polymer is a cellulose derivative and the composition has a viscosity of at least about 40cps.

4. A pharmaceutical composition comprising an effective amount of TSH and an effective amount of pharmaceutically acceptable polymer wherein the polymer is a cellulose derivative and the composition provides a serum T₃ level of no more than 2.5ng/ml over a 48-hour period after administration to a patient in need thereof.

5. A pharmaceutical composition comprising an effective amount of TSH and an effective amount of a pharmaceutically acceptable polymer wherein the polymer is a cellulose derivative and the composition provides an effective Tₘₐₓ of at least six hours after administration to a patient in need thereof.

6. A pharmaceutical composition comprising an effective amount of TSH and an effective amount of a pharmaceutically acceptable polymer wherein the polymer is a cellulose derivative and the composition provides an effective Cₘₐₓ in serum greater than about 2.0 mIU/L after administration to a patient in need thereof.

7. The pharmaceutical composition of any preceding claim wherein the TSH is TSH isolated from a mammal, or is recombinant mammalian TSH.

8. The pharmaceutical composition of any preceding claim wherein the cellulose derivative is selected from the group consisting of a methy cellulose, hydroxymethylcellulose, hydroxyethylcellulose, hydroxypropyl cellulose, hydroxypropyl methylcellulose, carboxymethylcellulose, sodium carboxymethylcellulose, ethyl hydroxyethyl cellulose, hypromellose, calcium carboxymethyl cellulose and a salt or ester of any of the foregoing.

9. The pharmaceutical composition of Claim 8, wherein the composition comprises 0.05% to 5% sodium carboxymethylcellulose.

10. The pharmaceutical composition of Claim 9, wherein the sodium carboxymethylcellulose has a molecular weight of 70,000 to 950,000.

11. The pharmaceutical composition of any preceding claim wherein the composition has a viscosity of at least 40 cps.

12. The pharmaceutical composition of Claim 11 wherein the composition has a viscosity of 40 to 125 cps.

13. Use of a composition of any preceding claim for the manufacture of a medicament for treating a thyroid condition.

14. The use of Claim 13, wherein the thyroid condition is selected from the group consisting of goiter and thyroid cancer.

15. Use of a composition of any of claims 1-12 in the manufacture of a medicament for maintaining blood plasma concentration of TSH above 2.0 mIU/L in a patient suffering from a thyroid condition.

16. A method for providing a modified-release formulation of TSH comprising admixing an effective amount of TSH and an effective amount of a pharmaceutically-acceptable polymer wherein the polymer is a cellulose derivative, thereby providing a modified-release formulation.

## Patentansprüche

1. Arzneimittel umfassend TSH und ein pharmazeutisch verträgliches Polymer, welches eine modifizierte Freisetzung des TSH in die Blutbahn eines Patienten ermöglicht, wobei das Polymer ein Cellulosederivat ist und, wenn es dem Patienten verabreicht wird, das Arzneimittel eine wirksame Tₘₐₓ von TSH im Serum des Patienten ergibt, welche mindestens etwa 20% länger ist als die wirksame Tₘₐₓ von TSH im Serum des Patienten, wenn eine entsprechende wässrige Lösung von TSH verabreicht wird.

2. Arzneimittel umfassend TSH und ein pharmazeutisch verträgliches Polymer, welches eine modifizierte Freisetzung des TSH in die Blutbahn eines Patienten ermöglicht, wobei das Polymer ein Cellulosederivat ist und, wenn es dem Patienten verabreicht wird, das Arzneimittel eine wirksame Cₘₐₓ von TSH im Serum des Patienten ergibt, welche mindestens etwa 20% niedriger ist als die wirksame Cₘₐₓ von TSH im Serum des Patienten, wenn eine entsprechende wässrige Lösung von TSH verabreicht wird.

3. Arzneimittel umfassend eine wirksame Menge von TSH und eine wirksame Menge eines pharmazeutisch verträglichen Polymers, wobei das Polymer ein Cellulosederivat ist und das Mittel eine Viskosität von mindestens etwa 40 cps hat.

4. Arzneimittel umfassend eine wirksame Menge von TSH und eine wirksame Menge eines pharmazeutisch verträglichen Polymers, wobei das Polymer ein Cellulosederivat ist und das Mittel über einen Zeitraum von 48h nach Verabreichung an einen Patienten, der dies benötigt, einen T₃-Serumspiegel von nicht mehr als 2,5 ng/ml ergibt.

5. Arzneimittel umfassend eine wirksame Menge von TSH und eine wirksame Menge eines pharmazeutisch verträglichen Polymers, wobei das Polymer ein Cellulosederivat ist und das Mittel eine wirksame Tₘₐₓ von mindestens sechs Stunden nach Verabreichung an einen Patienten, der dies benötigt, erbringt.

6. Arzneimittel umfassend eine wirksame Menge von TSH und eine wirksame Menge eines pharmazeutisch verträglichen Polymers, wobei das Polymer ein Cellulosederivat ist und das Mittel eine wirksame Cₘₐₓ im Serum erbringt, welche größer ist als etwa 2,0 mIU/l, nach Verabreichung an einen Patienten, der dies benötigt.

7. Arzneimittel nach einem der vorangehenden Ansprüche wobei das TSH ein aus einem Säuger isoliertes TSH oder ein rekombinantes Säuger-TSH ist.

8. Arzneimittel nach einem der vorangehenden Ansprüche wobei das Cellulosederivat ausgewählt ist aus der Gruppe bestehend aus Methylcellulose, Hydroxymethylcellulose, Hydroxyethylcellulose, Hydroxpropylcellulose, Hydroxypropylmethylcellulose, Carboxymethylcellulose, Natriumcarboxymethylcellulose, Ethylhydroxyethylcellulose, Hypromellose, Calciumcarboxymethylcellulose und einem Salz oder Ester von einem der vorstehenden.

9. Arzneimittel nach Anspruch 8, wobei das Mittel 0,05% bis 5% Natriumcarboxymethylcellulose umfasst.

10. Arzneimittel nach Anspruch 9, wobei die Natriumcarboxymethylcellulose ein Molekulargewicht von 70.000 bis 950.000 hat.

11. Arzneimittel nach einem der vorangehenden Ansprüche, wobei das Mittel eine Viskosität von mindestens 40 cps hat.

12. Arzneimittel nach Anspruch 11, wobei das Mittel eine Viskosität von 40 bis 125 cps hat.

13. Verwendung eines Arzneimittels nach einem der vorangehenden Ansprüche zur Herstellung eines Medikamentes für die Behandlung eines Schilddrüsenzustandes.

14. Verwendung nach Anspruch 13, wobei der Schilddrüsenzustand ausgewählt ist aus der Gruppe bestehend aus Kropf und Schilddrüsenkrebs.

15. Verwendung eines Mittels nach einem der Ansprüche 1 bis 12 zur Herstellung eines Medikamentes, um in einem Patienten, welcher an einem Schilddrüsenzustand leidet, eine Blutplasmakonzentration von TSH von über 2,0 mIU/L aufrechtzuerhalten.

16. Verfahren zur Bereitstellung einer Formulierung zur modifizierten Freisetzung von TSH, umfassend das Beimischen einer wirksamen Menge von TSH und einer wirksamen Menge eines pharmazeutisch verträglichen Polymers, wobei das Polymer ein Cellulosederivat ist, so dass eine Formulierung zur modifizierten Freisetzung bereitgestellt wird.

## Revendications

1. Composition pharmaceutique comprenant de la TSH et un polymère pharmaceutiquement acceptable qui permet la libération modifiée de la TSH dans le courant sanguin d'un patient, dans laquelle le polymère est un dérivé de cellulose et, lors de l'administration au patient, la composition pharmaceutique fournit une Tₘₐₓ efficace de TSH dans le sérum du patient qui est supérieure d'au moins 20 % à une Tₘₐₓ efficace de TSH dans le sérum du patient lorsqu'une solution aqueuse de TSH correspondante est administrée.

2. Composition pharmaceutique comprenant de la TSH et un polymère pharmaceutiquement acceptable qui permet la libération modifiée de la TSH dans le courant sanguin d'un patient, dans laquelle le polymère est un dérivé de cellulose et, lors de l'administration au patient, la composition pharmaceutique fournit une Cₘₐₓ efficace de TSH dans le sérum du patient qui est inférieure d'au moins 20 % à une Cₘₐₓ efficace de TSH dans le sérum du patient lorsqu'une solution aqueuse de TSH correspondante est administrée.

3. Composition pharmaceutique comprenant une quantité efficace de TSH et une quantité efficace d'un polymère pharmaceutiquement acceptable, dans laquelle le polymère est un dérivé de cellulose et la composition a une viscosité d'au moins environ 40 cps.

4. Composition pharmaceutique comprenant une quantité efficace de TSH et une quantité efficace d'un polymère pharmaceutiquement acceptable, dans laquelle le polymère est un dérivé de cellulose et la composition fournit un taux sérique de T₃ non supérieur à 2,5 ng/ml en une période de 48 heures après administration à un patient en ayant besoin.

5. Composition pharmaceutique comprenant une quantité efficace de TSH et une quantité efficace d'un polymère pharmaceutiquement acceptable, dans laquelle le polymère est un dérivé de cellulose et la composition fournit une Tₘₐₓ efficace d'au moins six heures après administration à un patient en ayant besoin.

6. Composition pharmaceutique comprenant une quantité efficace de TSH et une quantité efficace d'un polymère pharmaceutiquement acceptable, dans laquelle le polymère est un dérivé de cellulose et la composition fournit une Cₘₐₓ efficace dans le sérum supérieure à environ 2,0 mUI/l après administration à un patient en ayant besoin.

7. Composition pharmaceutique suivant l'une quelconque des revendications précédentes, dans laquelle la TSH est la TSH isolée d'un mammifère, ou bien est une TSH recombinante de mammifère.

8. Composition pharmaceutique suivant l'une quelconque des revendications précédentes, dans laquelle le dérivé de cellulose est choisi dans le groupe consistant en une méthylcellulose, une hydroxyméthylcellulose, une hydroxyéthylcellulose, une hydroxypropylcellulose, une hydroxypropylméthylcellulose, la carboxyméthylcellulose, la carboxyméthylcellulose sodique, l'éthylhydroxyéthylcellulose, l'hypromellose, la carboxyméthylcellulose calcique et un sel ou ester de n'importe lequel des dérivés précités.

9. Composition pharmaceutique suivant la revendication 8, ladite composition comprenant 0,05 % à 5 % de carboxyméthylcellulose sodique.

10. Composition pharmaceutique suivant la revendication 9, dans laquelle la carboxyméthylcellulose sodique a un poids moléculaire de 70 000 à 950 000.

11. Composition pharmaceutique suivant l'une quelconque des revendications précédentes, ladite composition ayant une viscosité d'au moins 40 cps.

12. Composition pharmaceutique suivant la revendication 11, ladite composition ayant une viscosité de 40 à 125 cps.

13. Utilisation d'une composition de l'une quelconque des revendications précédentes, pour la production d'un médicament pour le traitement d'une affection thyroïdienne.

14. Utilisation suivant la revendication 13, dans laquelle l'affection thyroïdienne est choisie dans le groupe consistant en le goitre et le cancer de la thyroïde.

15. Utilisation d'une composition de l'une quelconque des revendications 1 à 12 dans la production d'un médicament pour le maintien d'une concentration dans le plasma sanguin de TSH supérieure à 2,0 mUI/l chez un patient souffrant d'une affection thyroïdienne.

16. Procédé pour fournir une formulation à libération modifiée de TSH, comprenant le mélange d'une quantité efficace de TSH et d'une quantité efficace d'un polymère pharmaceutiquement acceptable, dans lequel le polymère est un dérivé de cellulose, ce qui donne une formulation à libération modifiée.
